# EUROPEAN PATENT APPLICATION

(11) **EP 4 494 654 A1**
(43) Date of publication of application: **22.01.2025**
(21) Application number: 23769780.0
(22) Date of filing: 14.03.2023
(51) Int. Cl.: A61K 39/395, C07K 16/28, C07H 19/12, A61P 35/00

(54) **PHARMACEUTICAL COMPOSITION OF ANTI-TIM-3 ANTIBODY AND HYPOMETHYLATING AGENT**

(30) Priority: 14.03.2022 CN 202210249527
(71) Applicant: Nanjing Shunxin Pharmaceuticals Co., Ltd. of Chiatai Tianqing Pharmaceutical Group, Nanjing, Jiangsu 211100 (CN); Chia Tai Tianqing Pharmaceutical Group Co., Ltd., Lianyungang City, Jiangsu 222062 (CN)
(72) Inventor: GUI, Zaizhi, Nanjing, Jiangsu 211100 (CN); YU, Ding, Nanjing, Jiangsu 211100 (CN); WANG, Xunqiang, Nanjing, Jiangsu 211100 (CN); XIAO, Ting, Nanjing, Jiangsu 211100 (CN); WANG, Liangliang, Nanjing, Jiangsu 211100 (CN)
(74) Representative: Lavoix
(86) International application number: PCT/CN2023/081360
(87) International publication number: WO 2023/174278

(57) **Abstract**

The present invention relates to a pharmaceutical composition of an anti-TIM-3 antibody and a hypomethylating agent. Preferably, the demethylating agent is azacitidine or decitabine. The present invention further provides a kit containing the pharmaceutical composition, a use of the pharmaceutical composition in the preparation of a drug for treating tumor, and a method of treating tumor by the pharmaceutical composition.

## Description

### TECHNICAL FIELD

The present disclosure belongs to the field of biomedicine, and particularly relates to a pharmaceutical combination of an anti-TIM-3 antibody and a demethylating drug.

### BACKGROUND

In recent years, tumors have shown increasing incidences, along with poor efficacy for malignancies and high metastasis rate in advanced diseases. At present, conventional therapies in the clinical use, such as radiotherapy, chemotherapy and surgery, can relieve the pain and prolong the survival period, but have limitations. Therefore, immunotherapy, which kills tumor cells by inhibiting immune checkpoints, re-activating immune cells, and avoiding immune escape of tumor cells, has gradually become a hotspot in the field of tumor treatment.

T cell immunoglobulin and mucin domain-containing protein 3 (TIM-3), also known as hepatitis A virus cellular receptor 2 (HAVCR2), is a member of the immunomodulatory protein TIM family (the human TIM family includes TIM-1, 3, and 4). TIM-3 is selectively expressed on the surface of activated Th1 cells, and is also expressed on marrow cells, DC cells, NK cells, and macrophages, as well as on various tumor cells, such as melanoma, cervical cancer and kidney cancer. TIM-3 has a number of different ligands including galectin9, phosphatidylserine (PtdSer), high mobility group box 1 (HMGB1), and carcinoembryonic antigen cell adhesion molecule 1 (CEACAM1). As an immune checkpoint, the physiological function of TIM-3 is to negatively regulate the immune function of an organism and avoid the damage of overly strong immune function or autoimmune function to the organism. More and more evidence indicate that TIM-3 protein and/or mRNA is up-regulated in various tumor tissues and tumor-associated immune cells, participates in tumor immune escape and immune response, and promotes tumor development.

DNA methylation is closely associated with the development of tumors. Almost all tumor cells are associated with DNA methylation abnormality, characterized by whole genome hypomethylation and regional hypermethylation. DNA methylation abnormality often causes inactivation of cancer suppressor genes, activation of proto-oncogenes, chromosome instability, gene mutation and miRNA expression disorder, thereby promoting the occurrence of tumors. The demethylating drug can inhibit DNA methyltransferase I (DNMTI) *in vivo* and *in vitro*, thereby inhibiting DNA methylation abnormality, activating expression of cancer suppressor genes, inducing tumor cell apoptosis, or inducing differentiation of tumor cells into normal cells.

However, single-drug treatment with anti-TIM-3 antibodies and demethylating drugs still has certain limitations, and there is still a need for alternative therapies, such as an anti-TIM-3 antibody in combination with a demethylating drug, to enhance the efficacy of tumor inhibition and potentially reduce toxicity.

### BRIEF SUMMARY

In one aspect, the present disclosure provides a pharmaceutical combination comprising an anti-TIM-3 antibody or an antigen-binding fragment thereof and a demethylating drug. In some embodiments, the demethylating drug is azacitidine or decitabine.

In some embodiments, the pharmaceutical combination comprises the anti-TIM-3 antibody or the antigen-binding fragment thereof in a unit dose of 60-1800 mg and azacitidine in a unit dose of 100 mg, 200 mg, and/or 300 mg. In some embodiments, the pharmaceutical combination comprises the anti-TIM-3 antibody or the antigen-binding fragment thereof in a unit dose of 60-1800 mg and decitabine in a unit dose of 10 mg, 25 mg, and/or 50 mg.

In some embodiments, the pharmaceutical combination comprises 100-1800 mg of the anti-TIM-3 antibody or the antigen-binding fragment thereof and 12-100 mg/m² or 200-300 mg of azacitidine. In some embodiments, the pharmaceutical combination comprises 100-1800 mg of the anti-TIM-3 antibody or the antigen-binding fragment thereof and 20-45 mg/m² of decitabine.

In some embodiments, the pharmaceutical combination is suitable for administration within a single treatment cycle and comprises 100-1800 mg of the anti-TIM-3 antibody or the antigen-binding fragment thereof and a demethylating drug. In some embodiments, the pharmaceutical combination comprises 100-1800 mg of the anti-TIM-3 antibody or the antigen-binding fragment thereof and 86-700 mg/m² or 1400-4200 mg of azacitidine. In some embodiments, the pharmaceutical combination comprises 100-1800 mg of the anti-TIM-3 antibody or the antigen-binding fragment thereof and 99-135 mg/m² of decitabine. In some embodiments, the pharmaceutical combination is used for treating a tumor.

In another aspect, the present disclosure provides a pharmaceutical combination for use in treating a tumor, comprising an anti-TIM-3 antibody or an antigen-binding fragment thereof and a demethylating drug; preferably, the demethylating drug is azacitidine or decitabine.

In another aspect, the present disclosure further provides a method for treating a tumor in a subject, comprising administering to the subject a therapeutically effective amount of the pharmaceutical combination of the present disclosure. In addition, the present disclosure further provides use of the pharmaceutical combination of the present disclosure for preparing a medicament for use in treating a tumor. Alternatively, the present disclosure further provides use of the anti-TIM-3 antibody or the antigen-binding fragment thereof and a demethylating drug for preparing a medicament for use in treating a tumor; preferably, the demethylating drug is azacitidine or decitabine. In some embodiments, the anti-TIM-3 antibody or the antigen-binding fragment thereof and the demethylating drug can be packaged separately or packaged together. Moreover, the anti-TIM-3 antibody or the antigen-binding fragment thereof can be packaged in a single unit or multiple units, and the demethylating drug can be packaged in a single unit or multiple units; preferably, the demethylating drug is azacitidine or decitabine.

In some embodiments, the anti-TIM-3 antibody or the antigen-binding fragment thereof and the demethylating drug can be packaged separately or packaged together. Moreover, the anti-TIM-3 antibody or the antigen-binding fragment thereof can be packaged in a single unit or multiple aliquots, and the demethylating drug can be packaged in a single unit or multiple aliquots; preferably, the demethylating drug is azacitidine or decitabine.

In some embodiments, the pharmaceutical combination is a fixed combination. In some embodiments, the fixed combination is present in the form of a solid pharmaceutical composition or a liquid pharmaceutical composition. In some embodiments, the pharmaceutical combination is a non-fixed combination. In some embodiments, the anti-TIM-3 antibody or the antigen-binding fragment thereof and the demethylating drug in the non-fixed combination are each present in the form of a pharmaceutical composition; preferably, the demethylating drug is azacitidine or decitabine.

In some embodiments, the anti-TIM-3 antibody or the antigen-binding fragment thereof and the demethylating drug can be administered simultaneously, sequentially, and/or alternately; preferably, the demethylating drug is azacitidine or decitabine.

In some embodiments, the anti-TIM-3 antibody or the antigen-binding fragment thereof is administered once every 1 week, every 2 weeks, every 3 weeks, or every 4 weeks. In some embodiments, the anti-TIM-3 antibody or the antigen-binding fragment thereof is administered at a dose of 100-1800 mg each time.

In some embodiments, the azacitidine is administered once daily at a dose of 12-100 mg/m², 37-75 mg/m², about 38 mg/m², about 50 mg/m², or about 75 mg/m² of azacitidine each time in an administration regimen of 7-day administration within a single treatment cycle.

In some embodiments, the azacitidine is administered once daily at a dose of about 200 mg or about 300 mg of azacitidine each time in an administration regimen of 14-day administration within a single treatment cycle.

In some embodiments, the decitabine is administered once daily at a dose of 20-45 mg/m² or about 20 mg/m² of decitabine each time in an administration regimen of consecutively 5-day administration within a single treatment cycle. Alternatively, the decitabine is administered three times daily at a dose of 11-15 mg/m², about 11 mg/m², or about 15 mg/m² of decitabine each time in an administration regimen of consecutively 3-day administration within a single treatment cycle.

In another aspect, the present disclosure provides a kit for use in treating a tumor, comprising the pharmaceutical combination of the present disclosure. In some embodiments, the kit comprises an anti-TIM-3 antibody or an antigen-binding fragment thereof and a demethylating drug; preferably, the demethylating drug is azacitidine or decitabine.

### BRIEF DESCRIPTION OF THE DRAWINGS

FIG. 1 shows the binding of h50B5 to CHO-S-HuTIM-3 cells highly expressing human TIM-3 detected by ELISA method.
FIG. 2 shows the promoting effect of h50B5 on IFN-γ secretion in MLR.
FIG. 3 shows the ADCC of h50B5 with Rituxan^{®} as the positive control.
FIG. 4 shows the CDC of h50B5 with Rituxan^{®} as the positive control.
FIG. 5 shows the plasma concentration curve of h50B5 after a single intravenous injection of h50B5 in human immune reconstruction mice (PBMC-NCG) with lung cancer HCC827 subcutaneous xenograft tumor.

### SUMMARY

The present disclosure provides a pharmaceutical combination comprising an anti-TIM-3 antibody or an antigen-binding fragment thereof and a demethylating drug. In some embodiments, the demethylating drug is azacitidine or decitabine. In some embodiments, the pharmaceutical combination is used for treating a tumor.

The present disclosure further provides use of the pharmaceutical combination of the present disclosure for preparing a medicament for use in treating a tumor. The present disclosure further provides use of the pharmaceutical combination of the present disclosure for treating a tumor. The present disclosure further provides a method for treating a tumor, comprising administering to a subject a therapeutically effective amount of the pharmaceutical combination of the present disclosure.

The present disclosure further provides a kit for use in treating a tumor, wherein the kit comprises the pharmaceutical combination of the present disclosure. In some embodiments, the kit further comprises an instruction for the pharmaceutical combination of the present disclosure in treating a tumor.

In addition, the present disclosure further provides an anti-TIM-3 antibody or an antigen-binding fragment thereof for use in treating a tumor. The present disclosure further provides use of the anti-TIM-3 antibody or the antigen-binding fragment thereof for preparing a medicament for use in treating a tumor. The present disclosure further provides use of the anti-TIM-3 antibody or the antigen-binding fragment thereof for treating a tumor. The present disclosure further provides a method for treating a tumor, comprising administering to a subject a therapeutically effective amount of the anti-TIM-3 antibody or the antigen-binding fragment thereof of the present disclosure.

In some embodiments, the tumor is a solid tumor and/or a non-solid tumor. In some embodiments, the tumor includes, but is not limited to, brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and/or lymphoma.

### Pharmaceutical combination

In one aspect, the present disclosure provides a pharmaceutical combination comprising an anti-TIM-3 antibody or an antigen-binding fragment thereof and a demethylating drug. In some embodiments, the pharmaceutical combination comprises the anti-TIM-3 antibody or the antigen-binding fragment thereof and azacitidine. In some embodiments, the pharmaceutical combination comprises the anti-TIM-3 antibody or the antigen-binding fragment thereof and decitabine.

In some embodiments, the pharmaceutical combination is packaged in the same kit further comprising an instruction for combined use of the anti-TIM-3 antibody or the antigen-binding fragment thereof and the demethylating drug in treating a tumor. In other embodiments, the anti-TIM-3 antibody or the antigen-binding fragment thereof and the demethylating drug in the pharmaceutical combination are packaged separately in their respective kits further comprising an instruction for combined use of the anti-TIM-3 antibody or the antigen-binding fragment thereof and the demethylating drug in treating a tumor.

In some embodiments, the pharmaceutical combination is packaged in the same kit further comprising an instruction for combined use of the anti-TIM-3 antibody or the antigen-binding fragment thereof and azacitidine in treating a tumor. In other embodiments, the anti-TIM-3 antibody or the antigen-binding fragment thereof and azacitidine in the pharmaceutical combination are packaged separately in their respective kits further comprising an instruction for combined use of the anti-TIM-3 antibody or the antigen-binding fragment thereof and azacitidine in treating a tumor.

In some embodiments, the pharmaceutical combination is packaged in the same kit further comprising an instruction for combined use of the anti-TIM-3 antibody or the antigen-binding fragment thereof and decitabine in treating a tumor. In other embodiments, the anti-TIM-3 antibody or the antigen-binding fragment thereof and decitabine in the pharmaceutical combination are packaged separately in their respective kits further comprising an instruction for combined use of the anti-TIM-3 antibody or the antigen-binding fragment thereof and decitabine in treating a tumor.

In some embodiments, the pharmaceutical combination is a fixed combination. In some embodiments, the fixed combination is present in the form of a solid pharmaceutical composition or a liquid pharmaceutical composition. In some embodiments, the pharmaceutical combination is a non-fixed combination. In some embodiments, the anti-TIM-3 antibody or the antigen-binding fragment thereof and the demethylating drug in the non-fixed combination are each present in the form of a pharmaceutical composition, and the pharmaceutical composition comprising the anti-TIM-3 antibody or the antigen-binding fragment thereof and the pharmaceutical composition comprising the demethylating drug are present or not present in the same sachet. In some embodiments, the anti-TIM-3 antibody or the antigen-binding fragment thereof and azacitidine in the non-fixed combination are each present in the form of a pharmaceutical composition, and the pharmaceutical composition comprising the anti-TIM-3 antibody or the antigen-binding fragment thereof and the pharmaceutical composition comprising azacitidine are present or not present in the same sachet. In some embodiments, the anti-TIM-3 antibody or the antigen-binding fragment thereof and decitabine in the non-fixed combination are each present in the form of a pharmaceutical composition, and the pharmaceutical composition comprising the anti-TIM-3 antibody or the antigen-binding fragment thereof and the pharmaceutical composition comprising decitabine are present or not present in the same sachet.

It is also an object of the present disclosure to at least provide a pharmaceutical pack comprising separately packaged pharmaceutical compositions in separate containers, wherein a pharmaceutical composition comprising an anti-TIM-3 antibody or an antigen-binding fragment thereof is contained in a first container, and a pharmaceutical composition comprising a demethylating drug (e.g., azacitidine or decitabine) is contained in a second container. In some specific embodiments, in the non-fixed combination, the pharmaceutical composition comprising the anti-TIM-3 antibody or the antigen-binding fragment thereof is a liquid formulation composition. In some specific embodiments, the liquid formulation composition is an injection.

In some specific embodiments, in the non-fixed combination, the pharmaceutical composition comprising azacitidine is a solid pharmaceutical composition. In some specific embodiments, the solid pharmaceutical composition is selected from the group consisting of tablets, capsules, powders, granules, pulvis, and the like, preferably powders or pulvis.

In some specific embodiments, in the non-fixed combination, the pharmaceutical composition comprising decitabine is a solid pharmaceutical composition. In some specific embodiments, the solid pharmaceutical composition is selected from the group consisting of tablets, capsules, powders, granules, pulvis, and the like, preferably powders or pulvis.

In some embodiments, the unit dose of the anti-TIM-3 antibody or the antigen-binding fragment thereof in the pharmaceutical combination is 60-1800 mg, 100-1600 mg, 120-1600 mg, 180-1200 mg, or 240-600 mg. In some embodiments, the unit dose of the anti-TIM-3 antibody or the antigen-binding fragment thereof in the pharmaceutical combination is about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, or about 1800 mg, or a range formed by any of the above values. In some embodiments, the unit dose of the anti-TIM-3 antibody or the antigen-binding fragment thereof in the pharmaceutical combination is about 240 mg, about 300 mg, about 360 mg, and/or about 600 mg. In some embodiments, the unit dose of the anti-TIM-3 antibody or the antigen-binding fragment thereof in the pharmaceutical combination is about 240 mg and/or about 600 mg.

In some embodiments, the unit dose of azacitidine in the pharmaceutical combination is 100 mg, 200 mg, and/or 300 mg.

In some embodiments, the unit dose of azacitidine in the pharmaceutical combination is 100 mg.

In some embodiments, the unit dose of azacitidine in the pharmaceutical combination is 200 mg and/or 300 mg. In some embodiments, the unit dose of decitabine in the pharmaceutical combination is 10 mg, 25 mg, and/or 50 mg.

In some embodiments, the pharmaceutical combination comprises the anti-TIM-3 antibody or the antigen-binding fragment thereof in a unit dose of 60-1800 mg, 100-1600 mg, 120-1600 mg, 180-1200 mg, or 240-600 mg and azacitidine in a unit dose of 100 mg. In some embodiments, the pharmaceutical combination comprises the anti-TIM-3 antibody or the antigen-binding fragment thereof in a unit dose of about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, or about 1800 mg, or a range formed by any of the above values and azacitidine in a unit dose of 100 mg. In some embodiments, the pharmaceutical combination comprises the anti-TIM-3 antibody or the antigen-binding fragment thereof in a unit dose of about 240 mg, about 300 mg, about 360 mg, and/or about 600 mg and azacitidine in a unit dose of 100 mg. In some embodiments, the pharmaceutical combination comprises the anti-TIM-3 antibody or the antigen-binding fragment thereof in a unit dose of about 240 mg and/or about 600 mg and azacitidine in a unit dose of 100 mg.

In some embodiments, the pharmaceutical combination comprises the anti-TIM-3 antibody or the antigen-binding fragment thereof in a unit dose of 60-1800 mg, 100-1600 mg, 120-1600 mg, 180-1200 mg, or 240-600 mg and azacitidine in a unit dose of 200 mg and/or 300 mg. In some embodiments, the pharmaceutical combination comprises the anti-TIM-3 antibody or the antigen-binding fragment thereof in a unit dose of about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, or about 1800 mg, or a range formed by any of the above values and azacitidine in a unit dose of 200 mg and/or 300 mg. In some embodiments, the pharmaceutical combination comprises the anti-TIM-3 antibody or the antigen-binding fragment thereof in a unit dose of about 240 mg, about 300 mg, about 360 mg, and/or about 600 mg and azacitidine in a unit dose of 200 mg and/or 300 mg. In some embodiments, the pharmaceutical combination comprises the anti-TIM-3 antibody or the antigen-binding fragment thereof in a unit dose of about 240 mg and/or about 600 mg and azacitidine in a unit dose of 200 mg and/or 300 mg.

In some embodiments, the pharmaceutical combination comprises the anti-TIM-3 antibody or the antigen-binding fragment thereof in a unit dose of 60-1800 mg, 100-1600 mg, 120-1600 mg, 180-1200 mg, or 240-600 mg and decitabine in a unit dose of 10 mg, 25 mg, and/or 50 mg. In some embodiments, the pharmaceutical combination comprises the anti-TIM-3 antibody or the antigen-binding fragment thereof in a unit dose of about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, or about 1800 mg, or a range formed by any of the above values and decitabine in a unit dose of 10 mg, 25 mg, and/or 50 mg. In some embodiments, the pharmaceutical combination comprises the anti-TIM-3 antibody or the antigen-binding fragment thereof in a unit dose of about 240 mg, about 300 mg, about 360 mg, and/or about 600 mg and decitabine in a unit dose of 10 mg, 25 mg, and/or 50 mg. In some embodiments, the pharmaceutical combination comprises the anti-TIM-3 antibody or the antigen-binding fragment thereof in a unit dose of 240 mg and/or about 600 mg and decitabine in a unit dose of 10 mg, 25 mg, and/or 50 mg.

In some embodiments, the anti-TIM-3 antibody or the antigen-binding fragment thereof may be a pharmaceutical composition comprising the anti-TIM-3 antibody or the antigen-binding fragment thereof. The pharmaceutical composition comprising the anti-TIM-3 antibody or the antigen-binding fragment thereof is in a single dose or in multiple doses, preferably in multiple doses.

In some embodiments, the azacitidine may be a pharmaceutical composition comprising azacitidine.

In some embodiments, the decitabine may be a pharmaceutical composition comprising decitabine.

In some embodiments, the pharmaceutical combination comprises 100-1800 mg, 600-1800 mg, 600-1600 mg, or 800-1600 mg of the anti-TIM-3 antibody or the antigen-binding fragment thereof. In some embodiments, the pharmaceutical combination comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof. In some embodiments, the pharmaceutical combination comprises about 800 mg, about 1200 mg, or about 1600 mg of the anti-TIM-3 antibody or the antigen-binding fragment thereof. In some embodiments, the pharmaceutical combination comprises about 1200 mg of the anti-TIM-3 antibody or the antigen-binding fragment thereof. In some embodiments, the pharmaceutical combination comprises about 1600 mg of the anti-TIM-3 antibody or the antigen-binding fragment thereof.

In some embodiments, the pharmaceutical combination comprises 12-100 mg/m², 37-75 mg/m², or 200-300 mg of azacitidine. In some embodiments, the pharmaceutical combination comprises about 12 mg/m² (such as about 12.375 mg/m²), about 19 mg/m² (such as about 18.75 mg/m²), about 25 mg/m² (such as about 24.75 mg/m² or about 25.125 mg/m²), about 28 mg/m² (such as about 28.125 mg/m²), about 38 mg/m² (such as about 37.5 mg/m²), about 50 mg/m² (such as about 50.25 mg/m²), about 56 mg/m² (such as about 56.25 mg/m²), about 75 mg/m², or about 100 mg/m², or a range formed by any of the above values of azacitidine. In some embodiments, the pharmaceutical combination comprises 37-75 mg/m² of azacitidine. In some embodiments, the pharmaceutical combination comprises about 38 mg/m², about 50 mg/m², or about 75 mg/m² of azacitidine. In some embodiments, the pharmaceutical combination comprises about 75 mg/m² of azacitidine. In some embodiments, the pharmaceutical combination comprises about 100 mg, about 120 mg, about 140 mg, about 160 mg, about 180 mg, about 200 mg, about 220 mg, about 240 mg, about 260 mg, about 280 mg, about 300 mg, about 320 mg, about 340 mg, about 360 mg, about 380 mg, or about 400 mg, or a range formed by any of the above values of azacitidine. In some embodiments, the pharmaceutical combination comprises about 200 mg or about 300 mg of azacitidine.

In some embodiments, the pharmaceutical combination comprises 20-45 mg/m², about 20 mg/m², about 33 mg/m², or about 45 mg/m² of decitabine. In some embodiments, the pharmaceutical combination comprises about 20 mg/m², about 33 mg/m², or about 45 mg/m², or a range formed by any of the above values of decitabine. In some embodiments, the pharmaceutical combination comprises 20-45 mg/m² of decitabine. In some embodiments, the pharmaceutical combination comprises about 20 mg/m², about 33 mg/m², or about 45 mg/m² of decitabine. In some embodiments, the pharmaceutical combination comprises about 20 mg/m² of decitabine.

In some embodiments, the pharmaceutical combination comprises 100-1800 mg, 600-1800 mg, 600-1600 mg, or 800-1600 mg of the anti-TIM-3 antibody or the antigen-binding fragment thereof and 12-100 mg/m², 37-75 mg/m², or 200-300 mg of azacitidine. In some embodiments, the pharmaceutical combination comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof, and about 12 mg/m² (such as about 12.375 mg/m²), about 19 mg/m² (such as about 18.75 mg/m²), about 25 mg/m² (such as about 24.75 mg/m² or about 25.125 mg/m²), about 28 mg/m² (such as about 28.125 mg/m²), about 38 mg/m² (such as about 37.5 mg/m²), about 50 mg/m² (such as about 50.25 mg/m²), about 56 mg/m² (such as about 56.25 mg/m²), about 75 mg/m², or about 100 mg/m², or a range formed by any of the above values of azacitidine. In some embodiments, the pharmaceutical combination comprises about 800 mg, about 1200 mg, or about 1600 mg of the anti-TIM-3 antibody or the antigen-binding fragment thereof and 37-75 mg/m² of azacitidine. In some embodiments, the pharmaceutical combination comprises about 800 mg, about 1200 mg, or about 1600 mg of the anti-TIM-3 antibody or the antigen-binding fragment thereof and about 38 mg/m², about 50 mg/m², or about 75 mg/m² of azacitidine. In some embodiments, the pharmaceutical combination comprises about 1200 mg of the anti-TIM-3 antibody or the antigen-binding fragment thereof and about 38 mg/m², about 50 mg/m², or about 75 mg/m² of azacitidine. In some embodiments, the pharmaceutical combination comprises about 1600 mg of the anti-TIM-3 antibody or the antigen-binding fragment thereof and about 38 mg/m², about 50 mg/m², or about 75 mg/m² of azacitidine. In some embodiments, the pharmaceutical combination comprises about 1200 mg of the anti-TIM-3 antibody or the antigen-binding fragment thereof and about 75 mg/m² of azacitidine. In some embodiments, the pharmaceutical combination comprises about 1600 mg of the anti-TIM-3 antibody or the antigen-binding fragment thereof and about 75 mg/m² of azacitidine. In some embodiments, the pharmaceutical combination comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof and about 200 mg, about 220 mg, about 240 mg, about 260 mg, about 280 mg, about 300 mg, or a range formed by any of the above values of azacitidine. In some embodiments, the pharmaceutical combination comprises about 800 mg, about 1200 mg, or about 1600 mg of the anti-TIM-3 antibody or the antigen-binding fragment thereof and about 200 mg or about 300 mg of azacitidine. In some embodiments, the pharmaceutical combination comprises about 1200 mg of the anti-TIM-3 antibody or the antigen-binding fragment thereof and about 200 mg or about 300 mg of azacitidine. In some embodiments, the pharmaceutical combination comprises about 1600 mg of the anti-TIM-3 antibody or the antigen-binding fragment thereof and about 200 mg or about 300 mg of azacitidine.

In some embodiments, the pharmaceutical combination comprises 100-1800 mg, 600-1800 mg, 600-1600 mg, or 800-1600 mg of the anti-TIM-3 antibody or the antigen-binding fragment thereof and 20-45 mg/m², about 20 mg/m², about 33 mg/m², or about 45 mg/m² of decitabine. In some embodiments, the pharmaceutical combination comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof and about 20 mg/m², about 33 mg/m², or about 45 mg/m², or a range formed by any of the above values of decitabine. In some embodiments, the pharmaceutical combination comprises about 800 mg, about 1200 mg, or about 1600 mg of the anti-TIM-3 antibody or the antigen-binding fragment thereof and 20-45 mg/m² of decitabine. In some embodiments, the pharmaceutical combination comprises about 800 mg, about 1200 mg, or about 1600 mg of the anti-TIM-3 antibody or the antigen-binding fragment thereof and about 20 mg/m², about 33 mg/m², or about 45 mg/m² of decitabine. In some embodiments, the pharmaceutical combination comprises about 1200 mg of the anti-TIM-3 antibody or the antigen-binding fragment thereof and about 20 mg/m², about 33 mg/m², or about 45 mg/m² of decitabine. In some embodiments, the pharmaceutical combination comprises about 1600 mg of the anti-TIM-3 antibody or the antigen-binding fragment thereof and about 20 mg/m², about 33 mg/m², or about 45 mg/m² of decitabine. In some embodiments, the pharmaceutical combination comprises about 1200 mg of the anti-TIM-3 antibody or the antigen-binding fragment thereof and about 20 mg/m² of decitabine. In some embodiments, the pharmaceutical combination comprises about 1600 mg of the anti-TIM-3 antibody or the antigen-binding fragment thereof and about 20 mg/m² of decitabine.

In some embodiments, in the pharmaceutical combination, the dose of the anti-TIM-3 antibody or the antigen-binding fragment thereof is a fixed dose in the pharmaceutical composition.

In some embodiments, in the pharmaceutical combination, the content of the anti-TIM-3 antibody or the antigen-binding fragment thereof is a daily dose.

In some embodiments, in the pharmaceutical combination, the content of the anti-TIM-3 antibody or the antigen-binding fragment thereof is a flat dose.

In some embodiments, in the pharmaceutical combination, the content of the anti-TIM-3 antibody or the antigen-binding fragment thereof is a dosage for one treatment cycle, each treatment cycle being 28 days.

In some embodiments, in the pharmaceutical combination, the content of azacitidine is a daily dose.

In some embodiments, in the pharmaceutical combination, the content of decitabine is a daily dose.

In some embodiments, the anti-TIM-3 antibody or the antigen-binding fragment thereof may be a pharmaceutical composition comprising the anti-TIM-3 antibody or the antigen-binding fragment thereof. The pharmaceutical composition comprising the anti-TIM-3 antibody or the antigen-binding fragment thereof is in a single dose or in multiple doses, preferably in multiple doses.

In some embodiments, the azacitidine may be a pharmaceutical composition comprising azacitidine.

In some embodiments, the decitabine may be a pharmaceutical composition comprising decitabine.

In some embodiments, the pharmaceutical combination is suitable for administration within a single treatment cycle and comprises 100-1800 mg, 600-1800 mg, 600-1600 mg, or 800-1600 mg of the anti-TIM-3 antibody or the antigen-binding fragment thereof. In some embodiments, the pharmaceutical combination is suitable for administration within a single treatment cycle and comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof. In some embodiments, the pharmaceutical combination is suitable for administration within a single treatment cycle and comprises about 800 mg, about 1200 mg, or about 1600 mg of the anti-TIM-3 antibody or the antigen-binding fragment thereof. In some embodiments, the pharmaceutical combination is suitable for administration within a single treatment cycle and comprises about 1200 mg of the anti-TIM-3 antibody or the antigen-binding fragment thereof. In some embodiments, the pharmaceutical combination is suitable for administration within a single treatment cycle and comprises about 1600 mg of the anti-TIM-3 antibody or the antigen-binding fragment thereof. In some embodiments, the pharmaceutical combination is suitable for administration within a single treatment cycle and comprises 86-700 mg/m², 262-525 mg/m², or 1400-4200 mg of azacitidine. In some embodiments, the pharmaceutical combination is suitable for administration within a single treatment cycle and comprises about 87 mg/m² (such as about 86.625 mg/m²), about 131 mg/m² (such as about 131.25 mg/m²), about 173 mg/m² (such as about 173.25 mg/m²), about 176 mg/m² (such as about 175.875 mg/m²), about 197 mg/m² (such as about 196.875 mg/m²), about 263 mg/m² (such as about 262.5 mg/m²), about 350 mg/m², about 352 mg/m² (such as about 351.75 mg/m²), about 394 mg/m² (such as about 393.75 mg/m²), about 525 mg/m², or about 700 mg/m², or a range formed by any of the above values of azacitidine. In some embodiments, the pharmaceutical combination is suitable for administration within a single treatment cycle and comprises 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, or 4200 mg, or a range formed by any of the above values of azacitidine. In some embodiments, the pharmaceutical combination is suitable for administration within a single treatment cycle and comprises about 263-525 mg/m² of azacitidine. In some embodiments, the pharmaceutical combination is suitable for administration within a single treatment cycle and comprises about 263 mg/m², about 350 mg/m², or about 525 mg/m² of azacitidine. In some embodiments, the pharmaceutical combination is suitable for administration within a single treatment cycle and comprises about 525 mg/m² of azacitidine. In some embodiments, the pharmaceutical combination is suitable for administration within a single treatment cycle and comprises 4200 mg of azacitidine.

In some embodiments, the pharmaceutical combination is suitable for administration within a single treatment cycle and comprises 99-135 mg/m², about 99 mg/m², about 100 mg/m², or about 135 mg/m² of decitabine. In some embodiments, the pharmaceutical combination is suitable for administration within a single treatment cycle and comprises 99-135 mg/m² of decitabine. In some embodiments, the pharmaceutical combination is suitable for administration within a single treatment cycle and comprises about 99 mg/m², about 100 mg/m², or about 135 mg/m² of decitabine. In some embodiments, the pharmaceutical combination is suitable for administration within a single treatment cycle and comprises about 100 mg/m² of decitabine.

In some embodiments, the pharmaceutical combination is suitable for administration within a single treatment cycle and comprises 100-1800 mg, 600-1800 mg, 600-1600 mg, or 800-1600 mg of the anti-TIM-3 antibody or the antigen-binding fragment thereof and 86-700 mg/m², 262-525 mg/m², 350-525 mg/m², or 1400-4200 mg of azacitidine. In some embodiments, the pharmaceutical combination is suitable for administration within a single treatment cycle and comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof, and about 87 mg/m² (such as about 86.625 mg/m²), about 131 mg/m² (such as about 131.25 mg/m²), about 173 mg/m² (such as about 173.25 mg/m²), about 176 mg/m² (such as about 175.875 mg/m²), about 197 mg/m² (such as about 196.875 mg/m²), about 263 mg/m² (such as about 262.5 mg/m²), about 350 mg/m², about 352 mg/m² (such as about 351.75 mg/m²), about 394 mg/m² (such as about 393.75 mg/m²), about 525 mg/m², or about 700 mg/m², or a range formed by any of the above values of azacitidine. In some embodiments, the pharmaceutical combination is suitable for administration within a single treatment cycle and comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof, and 1400 mg, 1500 mg, 1600 mg, 1700 mg, 1800 mg, 1900 mg, 2000 mg, 2100 mg, 2200 mg, 2300 mg, 2400 mg, 2500 mg, 2600 mg, 2700 mg, 2800 mg, 2900 mg, 3000 mg, 3100 mg, 3200 mg, 3300 mg, 3400 mg, 3500 mg, 3600 mg, 3700 mg, 3800 mg, 3900 mg, 4000 mg, 4100 mg, or 4200 mg, or a range formed by any of the above values of azacitidine. In some embodiments, the pharmaceutical combination is suitable for administration within a single treatment cycle and comprises about 800 mg, about 1200 mg, or about 1600 mg of the anti-TIM-3 antibody or the antigen-binding fragment thereof and 263-525 mg/m² of azacitidine. In some embodiments, the pharmaceutical combination is suitable for administration within a single treatment cycle and comprises about 800 mg, about 1200 mg, or about 1600 mg of the anti-TIM-3 antibody or the antigen-binding fragment thereof and about 263 mg/m², about 350 mg/m², or about 525 mg/m² of azacitidine. In some embodiments, the pharmaceutical combination is suitable for administration within a single treatment cycle and comprises about 1200 mg of the anti-TIM-3 antibody or the antigen-binding fragment thereof and about 525 mg/m² of azacitidine. In some embodiments, the pharmaceutical combination is suitable for administration within a single treatment cycle and comprises about 1600 mg of the anti-TIM-3 antibody or the antigen-binding fragment thereof and about 525 mg/m² of azacitidine. In some embodiments, the pharmaceutical combination is suitable for administration within a single treatment cycle and comprises about 800 mg, about 1200 mg, or about 1600 mg of the anti-TIM-3 antibody or the antigen-binding fragment thereof and 4200 mg of azacitidine. In some embodiments, the pharmaceutical combination is suitable for administration within a single treatment cycle and comprises about 1200 mg of the anti-TIM-3 antibody or the antigen-binding fragment thereof and 4200 mg of azacitidine. In some embodiments, the pharmaceutical combination is suitable for administration within a single treatment cycle and comprises about 1600 mg of the anti-TIM-3 antibody or the antigen-binding fragment thereof and 4200 mg of azacitidine.

In some embodiments, the pharmaceutical combination is suitable for administration within a single treatment cycle and comprises 100-1800 mg, 600-1800 mg, 600-1600 mg, or 800-1600 mg of the anti-TIM-3 antibody or the antigen-binding fragment thereof and 99-135 mg/m², about 99 mg/m², about 100 mg/m², or about 135 mg/m² of decitabine. In some embodiments, the pharmaceutical combination is suitable for administration within a single treatment cycle and comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof and 99-135 mg/m² of decitabine. In some embodiments, the pharmaceutical combination is suitable for administration within a single treatment cycle and comprises about 800 mg, about 1200 mg, or about 1600 mg of the anti-TIM-3 antibody or the antigen-binding fragment thereof and about 99 mg/m², about 100 mg/m², or about 135 mg/m² of decitabine. In some embodiments, the pharmaceutical combination is suitable for administration within a single treatment cycle and comprises about 1200 mg of the anti-TIM-3 antibody or the antigen-binding fragment thereof and about 100 mg/m² of decitabine. In some embodiments, the pharmaceutical combination is suitable for administration within a single treatment cycle and comprises about 1600 mg of the anti-TIM-3 antibody or the antigen-binding fragment thereof and about 100 mg/m² of decitabine.

In some embodiments, the anti-TIM-3 antibody or the antigen-binding fragment thereof may be a pharmaceutical composition comprising the anti-TIM-3 antibody or the antigen-binding fragment thereof. The pharmaceutical composition comprising the anti-TIM-3 antibody or the antigen-binding fragment thereof is in a single dose or in multiple doses, preferably in multiple doses.

In some embodiments, the azacitidine may be a pharmaceutical composition comprising azacitidine.

In some embodiments, the decitabine may be a pharmaceutical composition comprising decitabine.

In some embodiments, in the pharmaceutical combination, the mass ratio of the anti-TIM-3 antibody or the antigen-binding fragment thereof to azacitidine is (0.01-30):1, (0.1-15):1, (0.1-10):1, (0.1-5):1, (0.5-5):1, (0.5-3):1, (0.1-0.5):1, or (1-3):1, wherein the anti-TIM-3 antibody or the antigen-binding fragment thereof and azacitidine can be packaged separately or packaged together. Moreover, the anti-TIM-3 antibody or the antigen-binding fragment thereof can be packaged in a single unit or multiple aliquots (e.g., 2 aliquots, 3 aliquots, 4 aliquots, 5 aliquots, 6 aliquots, 7 aliquots, 8 aliquots, or more aliquots), and azacitidine can be packaged in a single unit or multiple aliquots.

In some embodiments, in the pharmaceutical combination, the mass ratio of the anti-TIM-3 antibody or the antigen-binding fragment thereof to decitabine is (0.1-60):1, (0.2-30):1, (2-15):1, (3-15):1, (3-12):1, or (6-12):1, wherein the anti-TIM-3 antibody or the antigen-binding fragment thereof and decitabine can be packaged separately or packaged together. Moreover, the anti-TIM-3 antibody or the antigen-binding fragment thereof can be packaged in a single unit or multiple aliquots (e.g., 2 aliquots, 3 aliquots, 4 aliquots, 5 aliquots, 6 aliquots, 7 aliquots, 8 aliquots, or more aliquots), and decitabine can be packaged in a single unit or multiple aliquots.

In some embodiments, the pharmaceutical combination comprises a pharmaceutical composition comprising the anti-TIM-3 antibody or the antigen-binding fragment thereof and a pharmaceutical composition comprising the demethylating drug, wherein the pharmaceutical composition comprising the anti-TIM-3 antibody or the antigen-binding fragment thereof is prepared as a single dose or multiple doses suitable for administering to a patient 100-1800 mg, 600-1800 mg, 600-1600 mg, or 800-1600 mg of the anti-TIM-3 antibody or the antigen-binding fragment thereof at the first administration. In some embodiments, the pharmaceutical combination comprises a pharmaceutical composition comprising the anti-TIM-3 antibody or the antigen-binding fragment thereof and a pharmaceutical composition comprising the demethylating drug, wherein the pharmaceutical composition comprising the anti-TIM-3 antibody or the antigen-binding fragment thereof is prepared as a single dose or multiple doses suitable for administering to a patient about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof at the first administration. In some embodiments, the pharmaceutical combination comprises a pharmaceutical composition comprising the anti-TIM-3 antibody or the antigen-binding fragment thereof and a pharmaceutical composition comprising the demethylating drug, wherein the pharmaceutical composition comprising the anti-TIM-3 antibody or the antigen-binding fragment thereof is prepared as a single dose or multiple doses suitable for administering to a patient about 800 mg, about 1200 mg, or about 1600 mg of the anti-TIM-3 antibody or the antigen-binding fragment thereof at the first administration, preferably multiple doses. In some embodiments, the pharmaceutical combination comprises a pharmaceutical composition comprising the anti-TIM-3 antibody or the antigen-binding fragment thereof and a pharmaceutical composition comprising the demethylating drug, wherein the pharmaceutical composition comprising the anti-TIM-3 antibody or the antigen-binding fragment thereof is prepared as a single dose or multiple doses suitable for administering to a patient about 1200 mg of the anti-TIM-3 antibody or the antigen-binding fragment thereof at the first administration, preferably multiple doses. In some embodiments, the pharmaceutical combination comprises a pharmaceutical composition comprising the anti-TIM-3 antibody or the antigen-binding fragment thereof and a pharmaceutical composition comprising the demethylating drug, wherein the pharmaceutical composition comprising the anti-TIM-3 antibody or the antigen-binding fragment thereof is prepared as a single dose or multiple doses suitable for administering to a patient about 1600 mg of the anti-TIM-3 antibody or the antigen-binding fragment thereof at the first administration, preferably multiple doses.

In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient 100-1800 mg, 600-1800 mg, 600-1600 mg, or 800-1600 mg of the anti-TIM-3 antibody or the antigen-binding fragment thereof at the first administration; the pharmaceutical composition comprising azacitidine is prepared as multiple doses suitable for administering to the patient daily 12-100 mg/m² or 37-75 mg/m² of azacitidine for a total of 7 days. In other embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient 100-1800 mg, 600-1800 mg, 600-1600 mg, or 800-1600 mg of the anti-TIM-3 antibody or the antigen-binding fragment thereof at the first administration; the pharmaceutical composition comprising azacitidine is prepared as multiple doses suitable for administering to the patient daily 200 mg or 300 mg of azacitidine for a total of 7-14 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof at the first administration; the pharmaceutical composition comprising azacitidine is prepared as multiple doses suitable for administering to the patient daily about 12 mg/m² (such as about 12.375 mg/m²), about 19 mg/m² (such as about 18.75 mg/m²), about 25 mg/m² (such as about 24.75 mg/m² or about 25.125 mg/m²), about 28 mg/m² (such as about 28.125 mg/m²), about 38 mg/m² (such as about 37.5 mg/m²), about 50 mg/m² (such as about 50.25 mg/m²), about 56 mg/m² (such as about 56.25 mg/m²), about 75 mg/m², or about 100 mg/m², or a range formed by any of the above values of azacitidine for a total of 7 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof at the first administration; the pharmaceutical composition comprising azacitidine is prepared as multiple doses suitable for administering to the patient daily 200 mg or 300 mg of azacitidine for a total of 7 days, 8 days, 9 days, 10 days, 11 days, 12 days, 13 days, or 14 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 800 mg, about 1200 mg, or about 1600 mg of the anti-TIM-3 antibody or the antigen-binding fragment thereof at the first administration; the pharmaceutical composition comprising azacitidine is prepared as multiple doses suitable for administering to the patient daily 37-75 mg/m² of azacitidine for a total of 7 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 800 mg, about 1200 mg, or about 1600 mg of the anti-TIM-3 antibody or the antigen-binding fragment thereof at the first administration; the pharmaceutical composition comprising azacitidine is prepared as multiple doses suitable for administering to the patient daily about 38 mg/m², about 50 mg/m², or about 75 mg/m² of azacitidine for a total of 7 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 1200 mg of the anti-TIM-3 antibody or the antigen-binding fragment thereof at the first administration; the pharmaceutical composition comprising azacitidine is prepared as multiple doses suitable for administering to the patient daily about 75 mg/m² of azacitidine for a total of 7 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 1600 mg of the anti-TIM-3 antibody or the antigen-binding fragment thereof at the first administration; the pharmaceutical composition comprising azacitidine is prepared as multiple doses suitable for administering to the patient daily about 75 mg/m² of azacitidine for a total of 7 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 800 mg, about 1200 mg, or about 1600 mg of the anti-TIM-3 antibody or the antigen-binding fragment thereof at the first administration; the pharmaceutical composition comprising azacitidine is prepared as multiple doses suitable for administering to the patient daily 300 mg of azacitidine for a total of 14 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 1200 mg of the anti-TIM-3 antibody or the antigen-binding fragment thereof at the first administration; the pharmaceutical composition comprising azacitidine is prepared as multiple doses suitable for administering to the patient daily 300 mg of azacitidine for a total of 14 days. In some embodiments, the pharmaceutical combination comprises a pharmaceutical composition comprising the anti-TIM-3 antibody or the antigen-binding fragment thereof and a pharmaceutical composition comprising azacitidine, wherein the pharmaceutical composition comprising the anti-TIM-3 antibody or the antigen-binding fragment thereof is prepared as a single dose or multiple doses suitable for administering to a patient about 1600 mg of the anti-TIM-3 antibody or the antigen-binding fragment thereof at the first administration; the pharmaceutical composition comprising azacitidine is prepared as multiple doses suitable for administering to the patient daily 300 mg of azacitidine for a total of 14 days.

In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient 100-1800 mg, 600-1800 mg, 600-1600 mg, or 800-1600 mg of the anti-TIM-3 antibody or the antigen-binding fragment thereof at the first administration; the pharmaceutical composition comprising decitabine is prepared as multiple doses suitable for administering to the patient daily 20-45 mg/m² of decitabine for 5 consecutive days, or the pharmaceutical composition comprising decitabine is prepared as multiple doses suitable for administering to the patient three times daily 11-15 mg/m² of decitabine each time for 3 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof at the first administration; the pharmaceutical composition comprising decitabine is prepared as multiple doses suitable for administering to the patient daily 20 mg/m² of decitabine for 5 consecutive days, or the pharmaceutical composition comprising decitabine is prepared as multiple doses suitable for administering to the patient three times daily 11 mg/m² or 15 mg/m² of decitabine each time for 3 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 800 mg, about 1200 mg, or about 1600 mg of the anti-TIM-3 antibody or the antigen-binding fragment thereof at the first administration; the pharmaceutical composition comprising decitabine is prepared as multiple doses suitable for administering to the patient daily about 20 mg/m² of decitabine for 5 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 1200 mg of the anti-TIM-3 antibody or the antigen-binding fragment thereof at the first administration; the pharmaceutical composition comprising decitabine is prepared as multiple doses suitable for administering to the patient daily 20 mg/m² of decitabine for 5 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 1600 mg of the anti-TIM-3 antibody or the antigen-binding fragment thereof at the first administration; the pharmaceutical composition comprising decitabine is prepared as multiple doses suitable for administering to the patient daily 20 mg/m² of decitabine for 5 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 800 mg, about 1200 mg, or about 1600 mg of the anti-TIM-3 antibody or the antigen-binding fragment thereof at the first administration; the pharmaceutical composition comprising decitabine is prepared as multiple doses suitable for administering to the patient three times daily 11 mg/m² or 15 mg/m² of decitabine each time for 3 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 1200 mg of the anti-TIM-3 antibody or the antigen-binding fragment thereof at the first administration; the pharmaceutical composition comprising decitabine is prepared as multiple doses suitable for administering to the patient three times daily 11 mg/m² or 15 mg/m² of decitabine each time for 3 consecutive days. In some embodiments, the pharmaceutical combination comprises a pharmaceutical composition comprising the anti-TIM-3 antibody or the antigen-binding fragment thereof and a pharmaceutical composition comprising decitabine, wherein the pharmaceutical composition comprising the anti-TIM-3 antibody or the antigen-binding fragment thereof is prepared as a single dose or multiple doses suitable for administering to a patient about 1600 mg of the anti-TIM-3 antibody or the antigen-binding fragment thereof at the first administration; the pharmaceutical composition comprising decitabine is prepared as multiple doses suitable for administering to the patient three times daily 11 mg/m² or 15 mg/m² of decitabine each time for 3 consecutive days.

In another aspect, the present disclosure further provides use of the anti-TIM-3 antibody or the antigen-binding fragment thereof and a demethylating drug for preparing a medicament for use in treating a tumor. The present disclosure further provides use of the pharmaceutical combination comprising the anti-TIM-3 antibody or the antigen-binding fragment thereof and a demethylating drug for treating a tumor. The present disclosure further provides a method for treating a tumor in a subject, comprising administering to the subject a therapeutically effective amount of the pharmaceutical combination comprising the anti-TIM-3 antibody or the antigen-binding fragment thereof and a demethylating drug. In some embodiments, the demethylating drug is azacitidine or decitabine.

In some embodiments, the present disclosure provides use of the anti-TIM-3 antibody or the antigen-binding fragment thereof and azacitidine for preparing a medicament for use in treating a tumor. The present disclosure further provides use of the pharmaceutical combination comprising the anti-TIM-3 antibody or the antigen-binding fragment thereof and azacitidine for treating a tumor. The present disclosure further provides a method for treating a tumor in a subject, comprising administering to the subject a therapeutically effective amount of the pharmaceutical combination comprising the anti-TIM-3 antibody or the antigen-binding fragment thereof and azacitidine.

In other embodiments, the present disclosure provides use of comprising the anti-TIM-3 antibody or the antigen-binding fragment thereof and decitabine for preparing a medicament for use in treating a tumor. The present disclosure further provides use of the pharmaceutical combination comprising the anti-TIM-3 antibody or the antigen-binding fragment thereof and decitabine for treating a tumor. The present disclosure further provides a method for treating a tumor in a subject, comprising administering to the subject a therapeutically effective amount of the pharmaceutical combination comprising the anti-TIM-3 antibody or the antigen-binding fragment thereof and decitabine.

In another aspect, the present disclosure provides a kit for use in treating a tumor, wherein the kit comprises a pharmaceutical composition comprising the anti-TIM-3 antibody or the antigen-binding fragment thereof and a pharmaceutical composition comprising a demethylating drug, and an instruction for combined use of the pharmaceutical composition comprising the anti-TIM-3 antibody or the antigen-binding fragment thereof and the pharmaceutical composition comprising the demethylating drug in treating a tumor.

In some embodiments, the present disclosure provides a kit for use in treating a tumor, wherein the kit comprises a pharmaceutical composition comprising the anti-TIM-3 antibody or the antigen-binding fragment thereof and a pharmaceutical composition comprising azacitidine, and an instruction for combined use of the pharmaceutical composition comprising the anti-TIM-3 antibody or the antigen-binding fragment thereof and the pharmaceutical composition comprising azacitidine in treating a tumor.

In other embodiments, the present disclosure provides a kit for use in treating a tumor, wherein the kit comprises a pharmaceutical composition comprising the anti-TIM-3 antibody or the antigen-binding fragment thereof and a pharmaceutical composition comprising decitabine, and an instruction for combined use of the pharmaceutical composition comprising the anti-TIM-3 antibody or the antigen-binding fragment thereof and the pharmaceutical composition comprising decitabine in treating a tumor.

In some embodiments, the tumor is a solid tumor and/or a non-solid tumor. In some embodiments, the tumor includes, but is not limited to, brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and lymphoma.

### Administration/treatment regimen of pharmaceutical combination

In some embodiments, in the above uses or methods of treatment, the anti-TIM-3 antibody or the antigen-binding fragment thereof and the demethylating drug can be administered simultaneously, sequentially, and/or alternately. In some embodiments, in the above uses or methods of treatment, the anti-TIM-3 antibody or the antigen-binding fragment thereof and the demethylating drug are each present in the form of a pharmaceutical composition, and can be administered simultaneously, sequentially, and/or alternately. In some embodiments, in the above uses or methods of treatment, the anti-TIM-3 antibody or the antigen-binding fragment thereof and the demethylating drug are each present in the form of a pharmaceutical composition, and are each administered intermittently. In some embodiments, in the above uses or methods of treatment, the anti-TIM-3 antibody or the antigen-binding fragment thereof and the demethylating drug are each administered according to the same or different administration regimens. In some embodiments, in the above uses or methods of treatment, the anti-TIM-3 antibody or the antigen-binding fragment thereof and the demethylating drug are each administered according to different administration regimens.

In some embodiments, in the above uses or methods of treatment, the anti-TIM-3 antibody or the antigen-binding fragment thereof and azacitidine are each present in the form of a pharmaceutical composition, and can be administered simultaneously, sequentially, and/or alternately. In some embodiments, in the above uses or methods of treatment, the anti-TIM-3 antibody or the antigen-binding fragment thereof and azacitidine are each present in the form of a pharmaceutical composition, and are each administered intermittently. In some embodiments, in the above uses or methods of treatment, the anti-TIM-3 antibody or the antigen-binding fragment thereof and azacitidine are each administered according to the same or different administration regimens. In some embodiments, in the above uses or methods of treatment, the anti-TIM-3 antibody or the antigen-binding fragment thereof and azacitidine are each administered according to different administration regimens.

In some embodiments, in the above uses or methods of treatment, the anti-TIM-3 antibody or the antigen-binding fragment thereof and decitabine are each present in the form of a pharmaceutical composition, and can be administered simultaneously, sequentially, and/or alternately. In some embodiments, in the above uses or methods of treatment, the anti-TIM-3 antibody or the antigen-binding fragment thereof and decitabine are each present in the form of a pharmaceutical composition, and are each administered intermittently. In some embodiments, in the above uses or methods of treatment, the anti-TIM-3 antibody or the antigen-binding fragment thereof and decitabine are each administered according to the same or different administration regimens. In some embodiments, in the above uses or methods of treatment, the anti-TIM-3 antibody or the antigen-binding fragment thereof and decitabine are each administered according to different administration regimens.

In some embodiments, in the above uses or methods of treatment, the anti-TIM-3 antibody or the antigen-binding fragment thereof is administered once every week (q1w), every 2 weeks (q2w), every 3 weeks (q3w), or every 4 weeks (q4w). In some specific embodiments, the anti-TIM-3 antibody or the antigen-binding fragment thereof is administered once every 3 weeks. In some specific embodiments, the anti-TIM-3 antibody or the antigen-binding fragment thereof is administered once every 4 weeks. In some embodiments, the anti-TIM-3 antibody or the antigen-binding fragment thereof is administered at a dose of 100-1800 mg, 600-1800 mg, 600-1600 mg, or 800-1600 mg each time. In some embodiments, the anti-TIM-3 antibody or the antigen-binding fragment thereof is administered at a dose of about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values each time. In some embodiments, the anti-TIM-3 antibody or the antigen-binding fragment thereof is administered at a dose of about 800 mg, about 1200 mg, or about 1600 mg each time. In some embodiments, the anti-TIM-3 antibody or the antigen-binding fragment thereof is administered at a dose of about 1200 mg each time. In some embodiments, the anti-TIM-3 antibody or the antigen-binding fragment thereof is administered at a dose of about 1600 mg each time. In some embodiments, the anti-TIM-3 antibody or the antigen-binding fragment thereof is administered once every 1 week, every 2 weeks, every 3 weeks, or every 4 weeks at a dose of 100-1800 mg, 600-1800 mg, 600-1600 mg, or 800-1600 mg of the anti-TIM-3 antibody or the antigen-binding fragment thereof each time. In some embodiments, the anti-TIM-3 antibody or the antigen-binding fragment thereof is administered once every 1 week, every 2 weeks, every 3 weeks, or every 4 weeks at a dose of 800-1600 mg of the anti-TIM-3 antibody or the antigen-binding fragment thereof each time. In some embodiments, the anti-TIM-3 antibody or the antigen-binding fragment thereof is administered once every 1 week, every 2 weeks, every 3 weeks, or every 4 weeks at a dose of about 800 mg, about 1200 mg, or about 1600 mg of the anti-TIM-3 antibody or the antigen-binding fragment thereof each time. In some embodiments, the anti-TIM-3 antibody or the antigen-binding fragment thereof is administered once every 4 weeks at a dose of about 800 mg, about 1200 mg, or about 1600 mg of the anti-TIM-3 antibody or the antigen-binding fragment thereof each time. In some embodiments, the anti-TIM-3 antibody or the antigen-binding fragment thereof is administered once every 4 weeks at a dose of about 1200 mg of the anti-TIM-3 antibody or the antigen-binding fragment thereof each time. In some embodiments, the anti-TIM-3 antibody or the antigen-binding fragment thereof is administered once every 4 weeks at a dose of about 1600 mg of the anti-TIM-3 antibody or the antigen-binding fragment thereof each time.

In some embodiments, in the above uses or methods of treatment, the azacitidine is administered once daily. In some embodiments, in the above uses or methods of treatment, the azacitidine is administered within a single treatment cycle for 7 days, 8 days, 9 days, 10 days, 11 days, 12 days, 13 days, or 14 days. In some embodiments, in the above uses or methods of treatment, the azacitidine is administered within a single treatment cycle for 7 days. In some embodiments, in the above uses or methods of treatment, the azacitidine is administered within a single treatment cycle for 14 days. In some embodiments, in the above uses or methods of treatment, the azacitidine is administered within a single treatment cycle for 7 consecutive days. In some embodiments, in the above uses or methods of treatment, the azacitidine is administered within a single treatment cycle for 14 consecutive days. In some embodiments, in the above uses or methods of treatment, the azacitidine is administered once daily within a single treatment cycle for 7 consecutive days. In some embodiments, in the above uses or methods of treatment, the azacitidine is administered once daily within a single treatment cycle for 14 consecutive days. In some embodiments, the azacitidine is administered at a dose of 12-100 mg/m² or 37-75 mg/m² each time. In some embodiments, the azacitidine is administered at a dose of about 12 mg/m² (such as about 12.375 mg/m²), about 19 mg/m² (such as about 18.75 mg/m²), about 25 mg/m² (such as about 24.75 mg/m² or about 25.125 mg/m²), about 28 mg/m² (such as about 28.125 mg/m²), about 38 mg/m² (such as about 37.5 mg/m²), about 50 mg/m² (such as about 50.25 mg/m²), about 56 mg/m² (such as about 56.25 mg/m²), about 75 mg/m², or about 100 mg/m² each time. In some embodiments, the azacitidine is administered at a dose of 200 mg or 300 mg each time. In some embodiments, the azacitidine is administered once daily at a dose of 12-100 mg/m² or 37-75 mg/m² of azacitidine each time in an administration regimen of 7-day administration within a single treatment cycle. In some embodiments, the azacitidine is administered once daily at a dose of about 38 mg/m², about 50 mg/m², or about 75 mg/m² of azacitidine each time in an administration regimen of 7-day administration within a single treatment cycle. In some embodiments, the azacitidine is administered once daily at a dose of about 38 mg/m², about 50 mg/m², or about 75 mg/m² of azacitidine each time in an administration regimen of consecutively 7-day administration within a single treatment cycle. In some embodiments, the azacitidine is administered once daily at a dose of about 300 mg of azacitidine each time in an administration regimen of consecutively 14-day administration within a single treatment cycle. The single treatment cycle of azacitidine is 4-6 weeks, preferably 4 weeks, 5 weeks, or 6 weeks, and more preferably 4 weeks. In some specific embodiments, the azacitidine is administered once daily at a dose of about 75 mg/m² of azacitidine each time in an administration regimen of consecutively 7-day administration and 21-day interruption. In another specific embodiment, the azacitidine is administered once daily at a dose of about 300 mg of azacitidine each time in an administration regimen of consecutively 14-day administration and 14-day interruption.

In some embodiments, in the above uses or methods of treatment, the decitabine is administered once daily. In some embodiments, in the above uses or methods of treatment, the decitabine is administered three times daily, preferably once every 8 hours. In some embodiments, in the above uses or methods of treatment, the decitabine is administered within a single treatment cycle for 5 days. In some embodiments, in the above uses or methods of treatment, the decitabine is administered within a single treatment cycle for 3 days. In some embodiments, in the above uses or methods of treatment, the decitabine is administered within a single treatment cycle for 5 consecutive days. In some embodiments, in the above uses or methods of treatment, the decitabine is administered within a single treatment cycle for 3 consecutive days. In some embodiments, the decitabine is administered at a dose of 20-45 mg/m² each time; preferably, the decitabine is administered at a dose of about 20 mg/m² each time. In some embodiments, the decitabine is administered at a dose of 11-15 mg/m² each time; preferably, the decitabine is administered at a dose of about 11 mg/m² or about 15 mg/m² each time. In some embodiments, the decitabine is administered once daily at a dose of about 20 mg/m² of decitabine each time in an administration regimen of consecutively 5-day administration within a single treatment cycle. In some embodiments, the decitabine is administered three times daily at a dose of 11-15 mg/m², about 11 mg/m², or about 15 mg/m² of decitabine each time in an administration regimen of consecutively 3-day administration within a single treatment cycle. In some embodiments, the single treatment cycle of decitabine is 4 weeks or more, preferably 4-6 weeks, and more preferably 4 weeks or 6 weeks. In some specific embodiments, the decitabine is administered once daily at a dose of about 20 mg/m² of decitabine each time in an administration regimen of consecutively 5-day administration and 23-day interruption. In other specific embodiments, the decitabine is administered three times daily at a dose of 11-15 mg/m², about 11 mg/m², or about 15 mg/m² of decitabine each time in an administration regimen of consecutively 3-day administration and 39-day interruption.

In some embodiments, the anti-TIM-3 antibody or the antigen-binding fragment thereof and the demethylating drug each have the same or different treatment cycles. In some specific embodiments, the anti-TIM-3 antibody or the antigen-binding fragment thereof and the demethylating drug have the same treatment cycle. For example, one treatment cycle is every 1 week, every 2 weeks, every 3 weeks, or every 4 weeks; preferably, one treatment cycle is every 4 weeks.

In some embodiments, the anti-TIM-3 antibody or the antigen-binding fragment thereof and azacitidine each have the same or different treatment cycles. In some specific embodiments, the anti-TIM-3 antibody or the antigen-binding fragment thereof and azacitidine have the same treatment cycle. For example, one treatment cycle is every 1 week, every 2 weeks, every 3 weeks, or every 4 weeks; preferably, one treatment cycle is every 4 weeks.

In some embodiments, the anti-TIM-3 antibody or the antigen-binding fragment thereof and decitabine each have the same or different treatment cycles. In some specific embodiments, the anti-TIM-3 antibody or the antigen-binding fragment thereof and decitabine have the same treatment cycle. For example, one treatment cycle is every 1 week, every 2 weeks, every 3 weeks, every 4 weeks, every 5 weeks, or every 6 weeks; preferably, one treatment cycle is every 4 weeks.

In some embodiments, in the above uses or methods of treatment, one treatment cycle is every 28 days, the anti-TIM-3 antibody or the antigen-binding fragment thereof is administered during each treatment cycle, and azacitidine is administered once daily for 7 days during each treatment cycle. In some embodiments, in the above uses or methods of treatment, one treatment cycle is every 28 days, the anti-TIM-3 antibody or the antigen-binding fragment thereof is administered once during each treatment cycle, and azacitidine is administered once daily for 7 days during each treatment cycle. In some embodiments, in the above uses or methods of treatment, one treatment cycle is every 28 days, the anti-TIM-3 antibody or the antigen-binding fragment thereof is administered on day 8 of each treatment cycle, and azacitidine is administered once daily for 7 days during each treatment cycle. In some embodiments, in the above uses or methods of treatment, one treatment cycle is every 28 days, the anti-TIM-3 antibody or the antigen-binding fragment thereof is administered once on day 8 of each treatment cycle, and azacitidine is administered once daily on days 1-7 of each treatment cycle.

In some embodiments, in the above uses or methods of treatment, one treatment cycle is every 28 days, the anti-TIM-3 antibody or the antigen-binding fragment thereof is administered during each treatment cycle, and azacitidine is administered once daily for 14 days during each treatment cycle. In some embodiments, in the above uses or methods of treatment, one treatment cycle is every 28 days, the anti-TIM-3 antibody or the antigen-binding fragment thereof is administered once during each treatment cycle, and azacitidine is administered once daily for 14 days during each treatment cycle. In some embodiments, in the above uses or methods of treatment, one treatment cycle is every 28 days, the anti-TIM-3 antibody or the antigen-binding fragment thereof is administered on day 8 of each treatment cycle, and azacitidine is administered once daily for 14 days during each treatment cycle. In some embodiments, in the above uses or methods of treatment, one treatment cycle is every 28 days, the anti-TIM-3 antibody or the antigen-binding fragment thereof is administered once on day 8 of each treatment cycle, and azacitidine is administered once daily on days 1-14 of each treatment cycle.

In some embodiments, in the above uses or methods of treatment, one treatment cycle is every 28 days, the anti-TIM-3 antibody or the antigen-binding fragment thereof is administered during each treatment cycle, and decitabine is administered once daily for 5 days during each treatment cycle. In some embodiments, in the above uses or methods of treatment, one treatment cycle is every 28 days, the anti-TIM-3 antibody or the antigen-binding fragment thereof is administered once during each treatment cycle, and decitabine is administered once daily for 5 days during each treatment cycle. In some embodiments, in the above uses or methods of treatment, one treatment cycle is every 28 days, the anti-TIM-3 antibody or the antigen-binding fragment thereof is administered on day 8 of each treatment cycle, and decitabine is administered once daily for 5 days during each treatment cycle. In some embodiments, in the above uses or methods of treatment, one treatment cycle is every 28 days, the anti-TIM-3 antibody or the antigen-binding fragment thereof is administered once on day 8 of each treatment cycle, and decitabine is administered once daily on days 1-5 of each treatment cycle.

In some embodiments, in the above uses or methods of treatment, one treatment cycle is every 28 days for the anti-TIM-3 antibody or the antigen-binding fragment thereof, and the anti-TIM-3 antibody or the antigen-binding fragment thereof is administered during each treatment cycle; one treatment cycle is every 42 days for decitabine, and decitabine is administered three times daily for 3 days during each treatment cycle. In some embodiments, in the above uses or methods of treatment, one treatment cycle is every 28 days for the anti-TIM-3 antibody or the antigen-binding fragment thereof, and the anti-TIM-3 antibody or the antigen-binding fragment thereof is administered once during each treatment cycle; one treatment cycle is every 42 days for decitabine, and decitabine is administered three times daily for 3 days during each treatment cycle. In some embodiments, in the above uses or methods of treatment, one treatment cycle is every 28 days for the anti-TIM-3 antibody or the antigen-binding fragment thereof, and the anti-TIM-3 antibody or the antigen-binding fragment thereof is administered on day 8 of each treatment cycle; one treatment cycle is every 42 days for decitabine, and decitabine is administered three times daily for 3 days during each treatment cycle. In some embodiments, in the above uses or methods of treatment, one treatment cycle is every 28 days for the anti-TIM-3 antibody or the antigen-binding fragment thereof, and the anti-TIM-3 antibody or the antigen-binding fragment thereof is administered once on day 8 of each treatment cycle; one treatment cycle is every 42 days for decitabine, and decitabine is administered three times daily on days 1-3 of each treatment cycle; preferably, decitabine is administered once every 8 hours on days 1-3 of each treatment cycle.

In some specific embodiments, in the above uses or methods of treatment, one treatment cycle is every 28 days, about 800 mg, about 1200 mg, or about 1600 mg of the anti-TIM-3 antibody or the antigen-binding fragment thereof is administered on day 8 of each treatment cycle, and 37-75 mg/m² of azacitidine is administered daily on days 1-7 of each treatment cycle. In some specific embodiments, in the above uses or methods of treatment, one treatment cycle is every 28 days, about 800 mg, about 1200 mg, or about 1600 mg of the anti-TIM-3 antibody or the antigen-binding fragment thereof is administered on day 8 of each treatment cycle, and about 38 mg/m², about 50 mg/m², or about 75 mg/m² of azacitidine is administered daily on days 1-7 of each treatment cycle. In some specific embodiments, in the above uses or methods of treatment, one treatment cycle is every 28 days, about 800 mg, about 1200 mg, or about 1600 mg of the anti-TIM-3 antibody or the antigen-binding fragment thereof is administered on day 8 of each treatment cycle, and about 75 mg/m² of azacitidine is administered daily on days 1-7 of each treatment cycle. In some specific embodiments, in the above uses or methods of treatment, one treatment cycle is every 28 days, about 1200 mg of the anti-TIM-3 antibody or the antigen-binding fragment thereof is administered on day 8 of each treatment cycle, and about 75 mg/m² of azacitidine is administered daily on days 1-7 of each treatment cycle. In some specific embodiments, in the above uses or methods of treatment, one treatment cycle is every 28 days, about 1600 mg of the anti-TIM-3 antibody or the antigen-binding fragment thereof is administered on day 8 of each treatment cycle, and about 75 mg/m² of azacitidine is administered daily on days 1-7 of each treatment cycle. In some specific embodiments, in the above uses or methods of treatment, one treatment cycle is every 28 days, about 800 mg, about 1200 mg, or about 1600 mg of the anti-TIM-3 antibody or the antigen-binding fragment thereof is administered on day 8 of each treatment cycle, and 300 mg of azacitidine is administered daily on days 1-14 of each treatment cycle. In some specific embodiments, in the above uses or methods of treatment, one treatment cycle is every 28 days, about 1200 mg of the anti-TIM-3 antibody or the antigen-binding fragment thereof is administered on day 8 of each treatment cycle, and 300 mg of azacitidine is administered daily on days 1-14 of each treatment cycle. In some specific embodiments, in the above uses or methods of treatment, one treatment cycle is every 28 days, about 1600 mg of the anti-TIM-3 antibody or the antigen-binding fragment thereof is administered on day 8 of each treatment cycle, and 300 mg of azacitidine is administered daily on days 1-14 of each treatment cycle.

In some specific embodiments, in the above uses or methods of treatment, one treatment cycle is every 28 days, about 800 mg, about 1200 mg, or about 1600 mg of the anti-TIM-3 antibody or the antigen-binding fragment thereof is administered on day 8 of each treatment cycle, and 20 mg/m² of decitabine is administered daily on days 1-5 of each treatment cycle. In some specific embodiments, in the above uses or methods of treatment, one treatment cycle is every 28 days, about 1200 mg of the anti-TIM-3 antibody or the antigen-binding fragment thereof is administered on day 8 of each treatment cycle, and 20 mg/m² of decitabine is administered daily on days 1-5 of each treatment cycle. In some specific embodiments, in the above uses or methods of treatment, one treatment cycle is every 28 days, about 1600 mg of the anti-TIM-3 antibody or the antigen-binding fragment thereof is administered on day 8 of each treatment cycle, and 20 mg/m² of decitabine is administered daily on days 1-5 of each treatment cycle.

In some specific embodiments, in the above uses or methods of treatment, one treatment cycle is every 28 days for the anti-TIM-3 antibody or the antigen-binding fragment thereof, and about 800 mg, about 1200 mg, or about 1600 mg of the anti-TIM-3 antibody or the antigen-binding fragment thereof is administered on day 8 of each treatment cycle; one treatment cycle is every 42 days for decitabine, decitabine is administered three times daily on days 1-3 of each treatment cycle, and 11-15 mg/m², about 11 mg/m², or about 15 mg/m² of decitabine is administered each time. In some specific embodiments, in the above uses or methods of treatment, one treatment cycle is every 28 days for the anti-TIM-3 antibody or the antigen-binding fragment thereof, and about 1200 mg of the anti-TIM-3 antibody or the antigen-binding fragment thereof is administered on day 8 of each treatment cycle; one treatment cycle is every 42 days for decitabine, decitabine is administered three times daily on days 1-3 of each treatment cycle, and 11-15 mg/m², about 11 mg/m², or about 15 mg/m² of decitabine is administered each time. In some specific embodiments, in the above uses or methods of treatment, one treatment cycle is every 28 days for the anti-TIM-3 antibody or the antigen-binding fragment thereof, and about 1600 mg of the anti-TIM-3 antibody or the antigen-binding fragment thereof is administered on day 8 of each treatment cycle; one treatment cycle is every 42 days for decitabine, decitabine is administered three times daily on days 1-3 of each treatment cycle, and 11-15 mg/m², about 11 mg/m², or about 15 mg/m² of decitabine is administered each time.

In some embodiments, the anti-TIM-3 antibody or the antigen-binding fragment thereof and azacitidine are administered to the subject at a mass ratio of (0.01-30):1, (0.1-15):1, (0.1-10):1, (0.1-5):1, (0.5-5):1, (0.5-3):1, (0.1-0.5):1 or (1-3):1 of the anti-TIM-3 antibody or the antigen-binding fragment thereof to azacitidine in each treatment cycle. In some embodiments, the anti-TIM-3 antibody or the antigen-binding fragment thereof and azacitidine are administered in multiple doses or a single dose. In some embodiments, the anti-TIM-3 antibody or the antigen-binding fragment thereof and azacitidine are both administered in multiple doses.

In some embodiments, the anti-TIM-3 antibody or the antigen-binding fragment thereof and decitabine are administered to the subject at a mass ratio of (0.1-60):1, (0.2-30):1, (2-15):1, (3-15):1, (3-12):1 or (6-12):1 of the anti-TIM-3 antibody or the antigen-binding fragment thereof to decitabine in each treatment cycle. In some embodiments, the anti-TIM-3 antibody or the antigen-binding fragment thereof and decitabine are administered in multiple doses or a single dose. In some embodiments, the anti-TIM-3 antibody or the antigen-binding fragment thereof and decitabine are administered in a single dose.

In some embodiments, in the above uses or methods of treatment, the anti-TIM-3 antibody or the antigen-binding fragment thereof may be administered to the subject at a dose selected from the group consisting of 0.01 to 50 mg/kg, 0.1 to 40 mg/kg, 0.1 to 35 mg/kg, 0.1 to 30 mg/kg, 0.1 to 25 mg/kg, 0.1 to 20 mg/kg, 0.1 to 15 mg/kg, 0.1 to 10 mg/kg, 1 to 40 mg/kg, 1 to 35 mg/kg, 1 to 30 mg/kg, 1 to 25 mg/kg, 1 to 20 mg/kg, 1 to 15 mg/kg, 1 to 10 mg/kg, 1 to 3 mg/kg, 3 to 40 mg/kg, 3 to 35 mg/kg, 3 to 30 mg/kg, 3 to 25 mg/kg, 3 to 20 mg/kg, 3 to 15 mg/kg, 3 to 10 mg/kg, 10 to 40 mg/kg, 10 to 30 mg/kg, 10 to 25 mg/kg, or 10 to 20 mg/kg; or the anti-TIM-3 antibody or the antigen-binding fragment thereof may be administered to the subject at a flat dose of 1 mg to 2400 mg, 20 mg to 1800 mg, 100 mg to 1800 mg, 300 mg to 1800 mg, 600 mg to 1600 mg, 700 mg to 1600 mg, 800 mg to 1600 mg, 900 mg to 1600 mg, 1000 mg to 1600 mg, 1100 mg to 1600 mg, 1200 mg to 1600 mg, 1300 mg to 1600 mg, 1400 mg to 1600 mg, 1500 mg to 1600 mg, 600 mg to 1500 mg, 800 mg to 1500 mg, 1000 mg to 1500 mg, 1200 mg to 1500 mg, 600 mg to 1200 mg, 800 mg to 1200 mg, 1200 mg to 1800 mg, 800 mg, 1000 mg, 1200 mg, 1600 mg or 1800 mg.

In some embodiments, in the above uses or methods of treatment, the regimen (e.g., dose and dosing cycle) of administering the anti-TIM-3 antibody or the antigen-binding fragment thereof and/or the demethylating drug (e.g., azacitidine or decitabine) may be adjusted according to the severity of the disease, the response to the disease, any treatment-related toxicity, and the age and health condition of a patient.

### Anti-TIM-3 antibody or antigen-binding fragment thereof

The anti-TIM-3 antibody or the antigen-binding fragment thereof described herein comprises heavy chain CDR1 (HCDR1) of the amino acid sequence set forth in SEQ ID NO: 1 or 11, HCDR2 of the amino acid sequence set forth in SEQ ID NO: 2 or 12, HCDR3 of the amino acid sequence set forth in SEQ ID NO: 3 or 13, light chain CDR1 (LCDR1) of the amino acid sequence set forth in SEQ ID NO: 4 or 14, LCDR2 of the amino acid sequence set forth in SEQ ID NO: 5 or 15, and LCDR3 of the amino acid sequence set forth in SEQ ID NO: 6 or 16. In some embodiments, the anti-TIM-3 antibody or the antigen-binding fragment thereof comprises HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6. In some embodiments, the anti-TIM-3 antibody or the antigen-binding fragment thereof comprises HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16. The above CDR sequences are shown in Table S1.

**Table S1. CDR sequences**

| | | |
|---|---|---|
| HCDR1 | GYSFTGYTIN | SEQ ID NO:1 |
| | GFNIKDYYMH | SEQ ID NO:11 |
| HCDR2 | LFNPYNGGTT | SEQ ID NO:2 |
| | WIDPENDNTIY | SEQ ID NO:12 |
| HCDR3 | RYYGYDAMDY | SEQ ID NO:3 |
| | ARDFGYVAWLVY | SEQ ID NO:13 |
| LCDR1 | KSSQSVLYSSNQKNHLA | SEQ ID NO:4 |
| | KASQNVDTAVA | SEQ ID NO:14 |
| LCDR2 | WASTRES | SEQ ID NO:5 |
| | SASNRYT | SEQ ID NO:15 |
| LCDR3 | HQYLSSYT | SEQ ID NO:6 |
| | QQYSSYPT | SEQ ID NO:16 |

It will be appreciated by those skilled in the art that, unless otherwise specified, the term "CDR" or "complementarity determining region" of a given antibody or a region thereof (e.g., a variable region) will be understood to encompass complementarity determining regions defined by any one of the known schemes. Although Table S1 has shown CDR sequences (where the CDR regions set forth in SEQ ID NOs: 1-6 are defined by the AbM numbering system), when reference is made to an antibody defined by a particular CDR sequence defined by certain numbering system of the present disclosure, the scope of the antibody also encompasses antibodies defined by CDR sequences that are converted to other arbitrary numbering system definitions (e.g., a combination of one or more of Kabat, Chothia, IMGT, or Contact, etc. definitions well known in the art). For example, the anti-TIM-3 antibodies or the antigen-binding fragments thereof comprising the following amino acid sequences are also encompassed within the scope of the anti-TIM-3 antibodies or the antigen-binding fragments thereof of the present disclosure: HCDR1 of the amino acid sequence set forth in SEQ ID NO: 1, HCDR2 of the amino acid sequence set forth in SEQ ID NO: 2, HCDR3 of the amino acid sequence set forth in ARRYYGYDAMDY (SEQ ID NO: 21), LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6.

In some embodiments, the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7 or 17, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8 or 18. In some embodiments, the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. In some embodiments, the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18. In some embodiments, the anti-TIM-3 antibody or the antigen-binding fragment thereof comprises a heavy chain variable region of the amino acid sequence set forth in SEQ ID NO: 7, and a light chain variable region of the amino acid sequence set forth in SEQ ID NO: 8. In some specific embodiments, the amino acid sequence of the heavy chain variable region of the anti-TIM-3 antibody or the antigen-binding fragment thereof is set forth in SEQ ID NO: 7, and the amino acid sequence of the light chain variable region is set forth in SEQ ID NO: 8. In some embodiments, the anti-TIM-3 antibody or the antigen-binding fragment thereof comprises a heavy chain variable region of the amino acid sequence set forth in SEQ ID NO: 17, and a light chain variable region of the amino acid sequence set forth in SEQ ID NO: 18. In some specific embodiments, the amino acid sequence of the heavy chain variable region of the anti-TIM-3 antibody or the antigen-binding fragment thereof is set forth in SEQ ID NO: 17, and the amino acid sequence of the light chain variable region is set forth in SEQ ID NO: 18.

QVQLVQSGAEVKKPGASVKVSCKASGYSFTGYTINWVRQAPGQGLEWMGLFNPYNGGTTYAQKFQGR
VTMTVDKSTSTVYMELSSLRSEDTAVYYCARRYYGYDAMDYWGQGTLVTVSS (SEQ ID NO:7);

DIQMTQSPSSLSASVGDRVTITCKSSQSVLYSSNQKNHLAWYQQKPGKAPKLLIYWASTRESGVPSRFSGS
GSGTDFTLTISSLQPEDFATYYCHQYLSSYTFGQGTKLEIK (SEQ ID NO:8);

EVQLQQSGAEVVRPGALVKLSCKASGFNIKDYYMHWVRQRPEQGLEWIGWIDPENDNTIYDPKFQDRA
SITADTSSNTAYLQLSSLTSEDTAVYYCARDFGYVAWLVYWGQGTLVTVSA (SEQ ID NO:17);

DIVMTQSQEFMSTSVGDRVSITCKASQNVDTAVAWYQQKPGQSPKLLIYSASNRYTGVPDRFTGTGSGTD
FTLTINNMQSEDLADYFCQQYSSYPTFGGRTKLEIK (SEQ ID NO:18).

In some embodiments, the anti-TIM-3 antibody or the antigen-binding fragment thereof may further comprise a constant region of an immunoglobulin, or a fragment, an analog, a variant, or a derivative of the constant region. In some embodiments, the heavy chain constant region is derived from a human immunoglobulin heavy chain, e.g., a heavy chain of IgG1, IgG2, IgG3, and IgG4, or other types of immunoglobulins, preferably a heavy chain of IgG4. In some embodiments, the light chain constant region is derived from a human immunoglobulin light chain, e.g., a κ light chain or a λ light chain of a human immunoglobulin. In some embodiments, the constant region may comprise modifications described in any text, e.g., insertion, deletion, substitution, or chemical modification of amino acids. In some embodiments, the constant region comprises a mutation that alters effector function. In some embodiments, any amino acid residue of the constant region may be substituted by an amino acid residue of any allotype.

In some embodiments, the anti-TIM-3 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 9 or 19, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 10 or 20. In some embodiments, the anti-TIM-3 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 9, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 10. In some embodiments, the anti-TIM-3 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20. In some embodiments, the anti-TIM-3 antibody or the antigen-binding fragment thereof comprises a heavy chain of the amino acid sequence set forth in SEQ ID NO: 9, and a light chain of the amino acid sequence set forth in SEQ ID NO: 10. In some specific embodiments, the amino acid sequence of the heavy chain of the anti-TIM-3 antibody or the antigen-binding fragment thereof is set forth in SEQ ID NO: 9, and the amino acid sequence of the light chain is set forth in SEQ ID NO: 10. In some embodiments, the anti-TIM-3 antibody or the antigen-binding fragment thereof comprises a heavy chain of the amino acid sequence set forth in SEQ ID NO: 19, and a light chain of the amino acid sequence set forth in SEQ ID NO: 20. In some specific embodiments, the amino acid sequence of the heavy chain of the anti-TIM-3 antibody or the antigen-binding fragment thereof is set forth in SEQ ID NO: 19, and the amino acid sequence of the light chain is set forth in SEQ ID NO: 20.

QVQLVQSGAEVKKPGASVKVSCKASGYSFTGYTINWVRQAPGQGLEWMGLFNPYNGGTTYAQKFQGR
VTMTVDKSTSTVYMELSSLRSEDTAVYYCARRYYGYDAMDYWGQGTLVTVSSASTKGPSVFPLAPCSRS
TSESTAALGCLVKDYFPEPVTVSWNSGALTSGVHTFPAVLQSSGLYSLSSVVTVPSSSLGTKTYTCNVDHK
PSNTKVDKRVESKYGPPCPPCPAPEFLGGPSVFLFPPKPKDTLMISRTPEVTCVVVDVSQEDPEVQFNWYV
DGVEVHNAKTKPREEQFNSTYRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFLYSRLTVDKSRWQ
EGNVFSCSVMHEALHNHYTQKSLSLSLGK (SEQ ID NO:9);

DIQMTQSPSSLSASVGDRVTITCKSSQSVLYSSNQKNHLAWYQQKPGKAPKLLIYWASTRESGVPSRFSGS
GSGTDFTLTISSLQPEDFATYYCHQYLSSYTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC (SEQ ID NO:10);

EVQLQQSGAEVVRPGALVKLSCKASGFNIKDYYMHWVRQRPEQGLEWIGWIDPENDNTIYDPKFQDRA
SITADTSSNTAYLQLSSLTSEDTAVYYCARDFGYVAWLVYWGQGTLVTVSAASTKGPSVFPLAPCSRSTSE
STAALGCLVKDYFPEPVTVSWNSGALTSGVHTFPAVLQSSGLYSLSSVVTVPSSSLGTKTYTCNVDHKPSN
TKVDKRVESKYGPPCPPCPAPEFLGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYSRLTVDKSRWQEG
NVFSCSVMHEALHNHYTQKSLSLSLGK (SEQ ID NO:19);

DIVMTQSQEFMSTSVGDRVSITCKASQNVDTAVAWYQQKPGQSPKLLIYSASNRYTGVPDRFTGTGSGTD
FTLTINNMQSEDLADYFCQQYSSYPTFGGRTKLEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNRG
EC (SEQ ID NO:20).

In some embodiments, the anti-TIM-3 antibody or the antigen-binding fragment thereof of the present disclosure is mAb 50B5 or an antigen-binding fragment thereof described in a patent application with patent publication NO. WO2020041520 or CN112566936. In other embodiments, the anti-TIM-3 antibody or the antigen-binding fragment thereof of the present disclosure is a chimeric antibody of mAb 50B5 or an antigen-binding fragment thereof. In other embodiments, the anti-TIM-3 antibody or the antigen-binding fragment thereof of the present disclosure is a humanized antibody of mAb 50B5 or an antigen-binding fragment thereof.

In other embodiments, the anti-TIM-3 antibody or the antigen-binding fragment thereof of the present disclosure is mAb 15B4 or an antigen-binding fragment thereof described in a patent application with patent publication NO. WO2020041520 or CN112566936. In other embodiments, the anti-TIM-3 antibody or the antigen-binding fragment thereof of the present disclosure is a chimeric antibody of mAb 15B4 or an antigen-binding fragment thereof. In other embodiments, the anti-TIM-3 antibody or the antigen-binding fragment thereof of the present disclosure is a humanized antibody of mAb 15B4 or an antigen-binding fragment thereof.

In other embodiments, the anti-TIM-3 antibody or the antigen-binding fragment thereof of the present disclosure is selected from the group consisting of sabotolimab, cobolimab, surzebiclimab, RG-7769 of Roche, SHR-1702 of Hengrui Pharmaceuticals, INCAGN-2390 of Agenus, BC-3402 of WuXi Biologics, LBL-003 of Leads Biolabs, LNL-005 of L&L Biopharma, or BMS-986258 of BMS.

The anti-TIM-3 antibody or the antigen-binding fragment thereof of the present disclosure exhibits a combination of one or several of the following properties: (a) binding to human TIM-3; (b) binding to monkey TIM-3; (d) stimulating IFN-γ secretion in PBMC; (e) having no ADCC; and (f) having no CDC.

In another aspect, the present disclosure provides an isolated nucleic acid molecule comprising the anti-TIM-3 antibody or the antigen-binding fragment thereof of the present disclosure.

In another aspect, the present disclosure provides an expression vector comprising the nucleic acid molecule of the present disclosure.

In another aspect, the present disclosure provides a host cell comprising the nucleic acid molecule of the present disclosure or the expression vector of the present disclosure.

The present disclosure further provides use of the anti-TIM-3 antibody or the antigen-binding fragment thereof of the present disclosure for preparing a medicament for use in treating a tumor. The present disclosure further provides use of the anti-TIM-3 antibody or the antigen-binding fragment thereof of the present disclosure for treating a tumor. The present disclosure further provides a method for treating a tumor, comprising administering to a subject a therapeutically effective amount of the anti-TIM-3 antibody or the antigen-binding fragment thereof of the present disclosure.

### Pharmaceutical composition comprising anti-TIM-3 antibody or antigen-binding fragment thereof

The anti-TIM-3 antibody or the antigen-binding fragment thereof is formulated for parenteral administration. In some embodiments, the anti-TIM-3 antibody or the antigen-binding fragment thereof is formulated for administration by intravenous, intramuscular, subcutaneous, or other parenteral routes, such as injection or infusion. In some embodiments, by intravenous, intramuscular, or subcutaneous administration. In some specific embodiments, by intravenous injection or infusion.

The anti-TIM-3 antibody or the antigen-binding fragment thereof can be formulated into suitable dosage forms including, but not limited to, tablet, troche, pill, capsule (e.g., hard capsule, soft capsule, enteric capsule, and microcapsule), elixir, granule, syrup, injection (intramuscular, intravenous, and intraperitoneal), granule, emulsion, suspension, solution, dispersant and dosage forms of sustained-released preparations for oral or non-oral administration. In some embodiments, the anti-TIM-3 antibody or the antigen-binding fragment thereof can be formulated into an injection.

In some embodiments, the unit dose of the pharmaceutical composition comprising the anti-TIM-3 antibody or the antigen-binding fragment thereof is 60-1800 mg, 100-1600 mg, 120-1600 mg, 180-1200 mg, 240-600 mg, or other amount of the anti-TIM-3 antibody or the antigen-binding fragment thereof, such as about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, and/or about 1800 mg, or a range formed by any of the above values of the anti-TIM-3 antibody or the antigen-binding fragment thereof.

In some embodiments, the pharmaceutical composition comprising the anti-TIM-3 antibody or the antigen-binding fragment thereof may further comprise one or more pharmaceutically acceptable carriers to make a suitable formulation. As used herein, the "pharmaceutically acceptable carrier" is used to describe any ingredients other than the compounds of the present disclosure, including excipients, diluents, encapsulating materials, fillers, buffering agents, or other agents. The selection of a pharmaceutically acceptable carrier will depend, to a large extent, on factors such as the particular mode of administration, the effect on solubility and stability, and the nature of the dosage form. As used herein, the "pharmaceutically acceptable carrier" includes any and all solvents, dispersion media, coatings, antibacterial and antifungal agents, isotonizing agents, absorption delaying agents, and the like that are physiologically compatible. Some examples of pharmaceutically acceptable carriers are water, saline, dextrose, glycerol, ethanol, and the like, and combinations thereof. In many cases, pharmaceutically acceptable carriers comprise isotonizing agents. Other examples of pharmaceutically acceptable carriers are wetting agents or minor amounts of auxiliary substances, such as wetting agents or emulsifiers, preservatives, or buffering agents, which enhance the shelf life or effectiveness of an antibody. In one specific embodiment, the pharmaceutical composition comprising the anti-TIM-3 antibody or the antigen-binding fragment thereof is a water-soluble injection, including but not limited to unlyophilized water-soluble formulations or water-soluble formulations obtained by reconstitution of lyophilized powders. In other embodiments, the pharmaceutical composition comprising the anti-TIM-3 antibody or the antigen-binding fragment thereof is a lyophilized formulation. The lyophilized formulation refers to a formulation prepared by subjecting an aqueous solution to a lyophilization process in which the substance is first frozen, and then the amount of the solvent is reduced by sublimation (primary drying process) and then by desorption (secondary drying process) until the amount of the solvent is reduced to a value that no longer supports the biological activity or a chemical reaction. The lyophilized formulation of the present disclosure can also be dried by other methods known in the art, such as spray drying and bubble drying.

### Azacitidine

As used in the present disclosure, the chemical name of azacitidine is 4-amino-1-β-D-ribofuranosyl-1,3,5-triazin-2(1H)-one, which has the structural formula of formula (I):

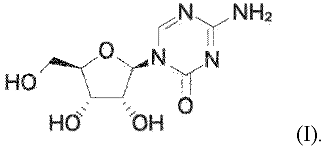

The dose or amount of azacitidine referred to in the present disclosure is based on the molecular weight of the compound of formula (I), unless otherwise indicated.

### Pharmaceutical composition comprising azacitidine

In some embodiments of the present disclosure, the unit dose of the pharmaceutical composition comprising azacitidine is 100 mg of azacitidine. In other embodiments, the unit dose of the pharmaceutical composition comprising azacitidine is 200 mg or 300 mg of azacitidine.

In some embodiments, the pharmaceutical composition comprising azacitidine is a formulation suitable for injection. In some embodiments, the pharmaceutical composition comprising azacitidine includes, but is not limited to, formulations suitable for oral, parenteral, and local administration. In some embodiments, the pharmaceutical composition comprising azacitidine is a formulation suitable for subcutaneous injection. In some embodiments, the pharmaceutical composition comprising azacitidine is a formulation suitable for oral administration.

In some embodiments, the pharmaceutical composition comprising azacitidine is a tablet, a powder, a troche, a pill, a capsule (e.g., a hard capsule, a soft capsule, an enteric capsule, and a microcapsule), an elixir, a granule, a syrup, an injection (subcutaneous, intramuscular, intravenous, and intraperitoneal), a granule, an emulsion, a suspension, a solution, a dispersant, and a sustained-released preparation for oral or non-oral administration. In some embodiments, the pharmaceutical composition comprising azacitidine is a solid formulation suitable for oral administration. In some embodiments, the pharmaceutical composition comprising azacitidine is a tablet. In some embodiments, the pharmaceutical composition comprising azacitidine is a solid formulation suitable for injection. In some embodiments, the pharmaceutical composition comprising azacitidine is a powder. In some embodiments, the pharmaceutical composition comprising azacitidine is a powder suitable for subcutaneous injection.

The pharmaceutical composition comprising azacitidine of the present disclosure can be manufactured using methods well known in the art, such as conventional mixing, dissolving, granulating, dragee-making, levigating, emulsifying, and lyophilizing.

A suitable formulation can be prepared by a conventional method using a pharmaceutically acceptable carrier well known in the art. The pharmaceutically acceptable carrier includes fillers, absorbents, wetting agents, binders, disintegrants, lubricants, and the like. In one specific embodiment, the suitable pharmaceutically acceptable carrier includes mannitol, hydrochloric acid, and/or sodium hydroxide. In one specific embodiment, the suitable pharmaceutically acceptable carrier includes croscarmellose sodium, magnesium stearate, mannitol, silicified microcrystalline cellulose, hydroxypropyl methylcellulose, lactose monohydrate, polyethylene glycol, titanium dioxide, and/or glyceryl triacetate.

### Decitabine

As used in the present disclosure, the chemical name of decitabine is 4-amino-1-(2-deoxy-β-D-erythro-ribofuranose)-1,3,5-triazin-2(1H)-one, which has the structural formula of formula (II):

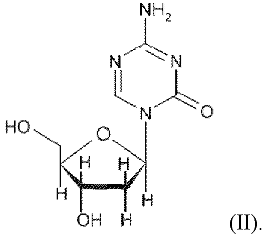

The dose or amount of decitabine referred to in the present disclosure is based on the molecular weight of the compound of formula (II), unless otherwise indicated.

### Pharmaceutical composition comprising decitabine

In some embodiments of the present disclosure, the unit dose of the pharmaceutical composition comprising decitabine is 10 mg-50 mg of decitabine. In some embodiments, the unit dose of the pharmaceutical composition comprising decitabine is 10 mg, 25 mg, or 50 mg of decitabine.

In some embodiments, the pharmaceutical composition comprising decitabine is a formulation suitable for injection. In some embodiments, the pharmaceutical composition comprising decitabine includes, but is not limited to, formulations suitable for oral, parenteral, and local administration. In some embodiments, the pharmaceutical composition comprising decitabine is a formulation suitable for intravenous injection.

In some embodiments, the pharmaceutical composition comprising decitabine is a tablet, a powder, a troche, a pill, a capsule (e.g., a hard capsule, a soft capsule, an enteric capsule, and a microcapsule), an elixir, a granule, a syrup, an injection (subcutaneous, intramuscular, intravenous, and intraperitoneal), a granule, an emulsion, a suspension, a solution, a dispersant, and a sustained-released preparation for oral or non-oral administration. In some embodiments, the pharmaceutical composition comprising decitabine is a solid formulation suitable for injection. In some embodiments, the pharmaceutical composition comprising decitabine is a powder. In some embodiments, the pharmaceutical composition comprising decitabine is a powder suitable for intravenous injection.

The pharmaceutical composition comprising decitabine of the present disclosure can be manufactured using methods well known in the art, such as conventional mixing, dissolving, granulating, dragee-making, levigating, emulsifying, and lyophilizing.

A suitable formulation can be prepared by a conventional method using a pharmaceutically acceptable carrier well known in the art. The pharmaceutically acceptable carrier includes fillers, absorbents, wetting agents, binders, disintegrants, lubricants, and the like. In one specific embodiment, the suitable pharmaceutically acceptable carrier includes potassium dihydrogen phosphate, hydrochloric acid, and/or sodium hydroxide.

### Mode of administration

The content below is not intended to limit the mode of administration of the pharmaceutical combination of the present disclosure.

The components in the pharmaceutical combination of the present disclosure can be administered independently, or some or all of the components are co-administered in various proper routes, including, but not limited to, oral administration or parenteral administration (e.g., by intravenous, intramuscular, local or subcutaneous routes). In some embodiments, the components in the pharmaceutical combination of the present disclosure can be administered independently, or some or all of the components are co-administered by oral administration or injection, for example, intravenous injection or subcutaneous injection.

The components in the pharmaceutical combination of the present disclosure can be independent suitable dosage forms, or some or all of the components are co-formulated in a suitable dosage form, including, but not limited to, tablet, troche, pill, capsule (for example, hard capsule, soft capsule, enteric capsule, and microcapsule), elixir, granule, syrup, injection (intramuscular, intravenous, intraperitoneal, and subcutaneous), granule, emulsion, suspension, solution, dispersant and dosage forms of sustained-released preparations for oral or non-oral administration.

The components in the pharmaceutical combination of the present disclosure can each independently contain a pharmaceutically acceptable carrier, or some or all of the components are co-formulated with a pharmaceutically acceptable carrier.

The pharmaceutical combination of the present disclosure may further comprise an additional therapeutic agent. In one embodiment, the additional therapeutic agent can be a known therapeutic agent for a tumor in the art.

### Tumor

In certain aspects, the tumor described herein is a malignant tumor (i.e., cancer); the malignant tumor refers to any malignant and/or invasive growth caused by abnormal cell growth. In some embodiments, the tumor is a non-solid tumor. In some embodiments, the tumor is a solid tumor. In some embodiments, the tumor is a refractory, unresectable, recurrent, advanced, and/or metastatic solid tumor. In some embodiments, the tumor is a refractory, recurrent, and/or advanced non-solid tumor.

In some embodiments, the tumor includes, but is not limited to, brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and lymphoma.

In some embodiments, the head and neck cancer is selected from the group consisting of head and neck squamous cell carcinoma (HNSCC), nasopharyngeal carcinoma, oral cancer, oropharyngeal cancer, laryngeal cancer, hypopharyngeal cancer, salivary gland cancer, sinus cancer, paranasal sinus cancer, and thyroid cancer.

In some embodiments, the lung cancer is selected from the group consisting of non-small cell lung cancer (NSCLC), small cell lung cancer, lung adenocarcinoma, and squamous cell carcinoma of lung.

In some embodiments, the melanoma is selected from the group consisting of advanced melanoma, unresectable melanoma, metastatic melanoma, melanoma with BRAF mutation, melanoma with NRAS mutation, cutaneous melanoma, and intraocular melanoma.

In some embodiments, the kidney cancer is renal cell carcinoma (RCC). In some embodiments, the renal cell carcinoma is selected from the group consisting of metastatic renal cell carcinoma, clear cell renal cell carcinoma (CCRCC), papillary renal cell carcinoma, chromophobe cell renal cell carcinoma, and tubular cystic renal cell carcinoma.

In some embodiments, the hematological malignancy is selected from the group consisting of myeloma, leukemia, myelodysplastic syndrome (MDS), myelofibrosis, and B-cell malignancy. In some embodiments, the leukemia is selected from the group consisting of acute lymphocytic leukemia (ALL), acute myelogenous leukemia (AML), acute promyelocytic leukemia (APL), chronic lymphocytic leukemia (CLL), chronic myelogenous leukemia (CML), and chronic myelomonocytic leukemia (CMML).

In some embodiments, the tumor is recurrent and/or refractory MDS. In some embodiments, the tumor is recurrent and/or refractory AML.

In some embodiments, the entity of the tumor has not previously been treated for the tumor (e.g., lacks an effective treatment regimen). In some embodiments, the entity of the tumor has not previously received chemotherapy to treat the tumor. In some embodiments, the entity of the tumor has not previously received first-line systemic chemotherapy to treat the tumor. In some embodiments, the entity of the tumor has not previously received surgery, radiation therapy, and/or immunotherapy to treat the tumor. In some embodiments, the entity of the tumor has not previously received adjuvant therapy to treat the tumor.

In some embodiments, the tumor is MDS, and the entity of the MDS has not previously been treated for MDS. In some embodiments, the tumor is MDS, and the entity of the MDS has not previously been treated for MDS and is intolerant to strong chemotherapy. In some embodiments, the tumor is MDS, and the entity of the MDS has not previously been treated for MDS, is suitable for treatment with a demethylating drug, and is intolerant to strong chemotherapy. In some embodiments, the tumor is AML, and the entity of the AML has not previously been treated for AML. In some embodiments, the tumor is AML, and the entity of the AML has not previously been treated for AML and is intolerant to strong chemotherapy. In some embodiments, the tumor is AML, and the entity of the AML has not previously been treated for AML, is suitable for treatment with a demethylating drug, and is intolerant to strong chemotherapy.

In some embodiments, the entity of the tumor has previously been treated for the tumor with one or more different anti-tumor therapies (e.g., drug treatment intolerance). In some embodiments, the tumor is MDS, and the entity of the MDS has previously been treated for MDS with one or more different anti-tumor therapies (e.g., drug treatment intolerance). In some embodiments, the tumor is recurrent and/or refractory MDS, and the entity of the recurrent and/or refractory MDS has previously been treated for MDS with one or more different anti-tumor therapies (e.g., drug treatment intolerance). In some embodiments, the tumor is AML, and the entity of the AML has previously been treated for AML with one or more different anti-tumor therapies (e.g., drug treatment intolerance). In some embodiments, the tumor is recurrent and/or refractory AML, and the entity of the recurrent and/or refractory AML has previously been treated for AML with one or more different anti-tumor therapies (e.g., drug treatment intolerance).

In some embodiments, the entity of the tumor has previously been treated for the tumor with one or more of surgery, radiation therapy, chemotherapy, and immunotherapy (e.g., treatment failure or inapplicability). In some embodiments, the tumor is MDS, and the entity of the MDS has previously been treated for MDS with one or more of surgery, radiation therapy, chemotherapy, and immunotherapy (e.g., treatment failure or inapplicability). In some embodiments, the tumor is recurrent and/or refractory MDS, and the entity of the recurrent and/or refractory MDS has previously been treated for MDS with one or more of surgery, radiation therapy, chemotherapy, and immunotherapy (e.g., treatment failure or inapplicability). In some embodiments, the tumor is AML, and the entity of the AML has previously been treated for AML with one or more of surgery, radiation therapy, chemotherapy, and immunotherapy (e.g., treatment failure or inapplicability). In some embodiments, the tumor is recurrent and/or refractory AML, and the entity of the recurrent and/or refractory AML has previously been treated for AML with one or more of surgery, radiation therapy, chemotherapy, and immunotherapy (e.g., treatment failure or inapplicability).

In some embodiments, the entity of the tumor has previously been treated for the tumor with chemotherapy (e.g., treatment failure or inapplicability to chemotherapy). In some embodiments, the tumor is MDS, and the entity of the MDS has previously been treated for MDS with chemotherapy (e.g., treatment failure or inapplicability to chemotherapy). In some embodiments, the tumor is recurrent and/or refractory MDS, and the entity of the recurrent and/or refractory MDS has previously been treated for MDS with chemotherapy (e.g., treatment failure or inapplicability to chemotherapy). In some embodiments, the tumor is AML, and the entity of the AML has previously been treated for AML with chemotherapy (e.g., treatment failure or inapplicability to chemotherapy). In some embodiments, the tumor is recurrent and/or refractory AML, and the entity of the recurrent and/or refractory AML has previously been treated for AML with chemotherapy (e.g., treatment failure or inapplicability to chemotherapy).

In some embodiments, the entity of the tumor has previously been treated for the tumor with a first-line systemic chemotherapy regimen (e.g., treatment failure or inapplicability to systemic chemotherapy). In some embodiments, the tumor is MDS, and the entity of the MDS has previously been treated for MDS with a first-line systemic chemotherapy regimen (e.g., treatment failure or inapplicability to systemic chemotherapy). In some embodiments, the tumor is recurrent and/or refractory MDS, and the entity of the recurrent and/or refractory MDS has previously been treated for MDS with a first-line systemic chemotherapy regimen (e.g., treatment failure or inapplicability to systemic chemotherapy). In some embodiments, the tumor is AML, and the entity of the AML has previously been treated for AML with a first-line systemic chemotherapy regimen (e.g., treatment failure or inapplicability to systemic chemotherapy). In some embodiments, the tumor is recurrent and/or refractory AML, and the entity of the recurrent and/or refractory AML has previously been treated for AML with a first-line systemic chemotherapy regimen (e.g., treatment failure or inapplicability to systemic chemotherapy).

In some embodiments, the entity of the tumor has been treated for other malignancies with surgery.

In some embodiments, the entity of the tumor is an AML patient with a FLT3 mutation. In some embodiments, the entity of the tumor has been treated with an FLT3 inhibitor (e.g., treatment failure).

The present disclosure further provides the following specific embodiments, which, however, are not intended to limit the scope of the present disclosure:
Embodiment 1. A pharmaceutical combination comprising an anti-TIM-3 antibody or an antigen-binding fragment thereof and a demethylating drug, wherein the anti-TIM-3 antibody or the antigen-binding fragment thereof comprises: HCDR1 of the amino acid sequence set forth in SEQ ID NO: 1 or 11, HCDR2 of the amino acid sequence set forth in SEQ ID NO: 2 or 12, HCDR3 of the amino acid sequence set forth in SEQ ID NO: 3 or 13, LCDR1 of the amino acid sequence set forth in SEQ ID NO: 4 or 14, LCDR2 of the amino acid sequence set forth in SEQ ID NO: 5 or 15, and LCDR3 of the amino acid sequence set forth in SEQ ID NO: 6 or 16; preferably,
   the anti-TIM-3 antibody or the antigen-binding fragment thereof comprises: HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6; or the anti-TIM-3 antibody or the antigen-binding fragment thereof comprises: HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16.
Embodiment 2. The pharmaceutical combination according to embodiment 1, wherein the anti-TIM-3 antibody or the antigen-binding fragment thereof comprises: a heavy chain variable region having an amino acid sequence having at least 95% identity to the amino acid sequence set forth in SEQ ID NO: 7 or 17, and a light chain variable region having an amino acid sequence having at least 95% identity to the amino acid sequence set forth in SEQ ID NO: 8 or 18; preferably,
   the anti-TIM-3 antibody or the antigen-binding fragment thereof comprises: a heavy chain variable region having an amino acid sequence having at least 95% identity to the amino acid sequence set forth in SEQ ID NO: 7, and a light chain variable region having an amino acid sequence having at least 95% identity to the amino acid sequence set forth in SEQ ID NO: 8; or the anti-TIM-3 antibody or the antigen-binding fragment thereof comprises: a heavy chain variable region having an amino acid sequence having at least 95% identity to the amino acid sequence set forth in SEQ ID NO: 17, and a light chain variable region having an amino acid sequence having at least 95% identity to the amino acid sequence set forth in SEQ ID NO: 18.
Embodiment 3. The pharmaceutical combination according to embodiment 2, wherein the anti-TIM-3 antibody or the antigen-binding fragment thereof comprises: a heavy chain having an amino acid sequence having at least 95% identity to the amino acid sequence set forth in SEQ ID NO: 9 or 19, and a light chain having an amino acid sequence having at least 95% identity to the amino acid sequence set forth in SEQ ID NO: 10 or 20; preferably,
   the anti-TIM-3 antibody or the antigen-binding fragment thereof comprises: a heavy chain having an amino acid sequence having at least 95% identity to the amino acid sequence set forth in SEQ ID NO: 9, and a light chain having an amino acid sequence having at least 95% identity to the amino acid sequence set forth in SEQ ID NO: 10; or the anti-TIM-3 antibody or the antigen-binding fragment thereof comprises: a heavy chain having an amino acid sequence having at least 95% identity to the amino acid sequence set forth in SEQ ID NO: 19, and a light chain having an amino acid sequence having at least 95% identity to the amino acid sequence set forth in SEQ ID NO: 20.
Embodiment 4. The pharmaceutical combination according to any one of embodiments 1-3, wherein the demethylating drug is azacitidine or decitabine.
Embodiment 5. The pharmaceutical combination according to any one of embodiments 1-4, wherein the unit dose of the anti-TIM-3 antibody or the antigen-binding fragment thereof is 60-1800 mg, 100-1600 mg, 120-1600 mg, 180-1200 mg, or 240-600 mg.
Embodiment 6. The pharmaceutical combination according to any one of embodiments 1-5, wherein the unit dose of azacitidine is 100 mg, 200 mg, and/or 300 mg.
Embodiment 7. The pharmaceutical combination according to any one of embodiments 1-6, wherein the mass ratio of the anti-TIM-3 antibody or the antigen-binding fragment thereof to azacitidine is (0.01-30):1, (0.1-15):1, (0.1-10):1, (0.1-5):1, (0.5-5):1, (0.5-3):1, (0.1-0.5):1, or (1-3):1.
Embodiment 8. The pharmaceutical combination according to any one of embodiments 1-5, wherein the unit dose of decitabine is 10 mg, 25 mg, and/or 50 mg.
Embodiment 9. The pharmaceutical combination according to any one of embodiments 1-5 or 8, wherein the mass ratio of the anti-TIM-3 antibody or the antigen-binding fragment thereof to decitabine is (0.1-60):1, (0.2-30):1, (2-15):1, (3-15):1, (3-12):1, or (6-12):1.
Embodiment 10. The pharmaceutical combination according to any one of embodiments 1-5, wherein the pharmaceutical combination comprises 100-1800 mg, 600-1800 mg, 600-1600 mg, or 800-1600 mg of the anti-TIM-3 antibody or the antigen-binding fragment thereof.
Embodiment 11. The pharmaceutical combination according to embodiment 10, wherein the pharmaceutical combination further comprises 12-100 mg/m², 37-75 mg/m², or 200-300 mg of azacitidine.
Embodiment 12. The pharmaceutical combination according to embodiment 10, wherein the pharmaceutical combination further comprises 20-45 mg/m² of decitabine.
Embodiment 13. The pharmaceutical combination according to any one of embodiments 1-5, wherein the pharmaceutical combination is suitable for administration within a single treatment cycle and comprises 100-1800 mg, 600-1800 mg, 600-1600 mg, or 800-1600 mg of the anti-TIM-3 antibody or the antigen-binding fragment thereof.
Embodiment 14. The pharmaceutical combination according to embodiment 13, wherein the pharmaceutical combination further comprises 86-700 mg/m², 262-525 mg/m², or 1400-4200 mg of azacitidine.
Embodiment 15. The pharmaceutical combination according to embodiment 13, wherein the pharmaceutical combination further comprises 99-135 mg/m² of decitabine.
Embodiment 16. The pharmaceutical combination according to any one of embodiments 1-15, wherein the pharmaceutical combination is used for treating a tumor.
Embodiment 17. The pharmaceutical combination according to embodiment 16, wherein the tumor is a solid tumor or a non-solid tumor.
Embodiment 18. The pharmaceutical combination according to embodiment 16, wherein the tumor is selected from the group consisting of brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and lymphoma.
Embodiment 19. The pharmaceutical combination of embodiment 18, wherein the hematological malignancy is selected from the group consisting of myeloma, leukemia, myelodysplastic syndrome, myelofibrosis, and B-cell malignancy.
Embodiment 20. Use of the pharmaceutical combination according to any one of embodiments 1-15 for preparing a medicament for use in treating a tumor.
Embodiment 21. The use according to embodiment 20, wherein the tumor is a solid tumor or a non-solid tumor.
Embodiment 22. The use according to embodiment 20, wherein the tumor is selected from the group consisting of brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and lymphoma.
Embodiment 23. The use according to embodiment 22, wherein the hematological malignancy is selected from the group consisting of myeloma, leukemia, myelodysplastic syndrome, myelofibrosis, and B-cell malignancy.
Embodiment 24. The use according to any one of embodiments 20-23, wherein the anti-TIM-3 antibody or the antigen-binding fragment thereof and the demethylating drug can be administered simultaneously, sequentially, and/or alternately.
Embodiment 25. The use according to embodiment 24, wherein the anti-TIM-3 antibody or the antigen-binding fragment thereof and azacitidine can be administered simultaneously, sequentially, and/or alternately.
Embodiment 26. The use according to embodiment 24, wherein the anti-TIM-3 antibody or the antigen-binding fragment thereof and decitabine can be administered simultaneously, sequentially, and/or alternately.
Embodiment 27. The use according to any one of embodiments 20-26, wherein the anti-TIM-3 antibody or the antigen-binding fragment thereof and the demethylating drug have the same treatment cycle, and one treatment cycle is every 1 week, every 2 weeks, every 3 weeks, or every 4 weeks; preferably, one treatment cycle is every 4 weeks.
Embodiment 28. The use according to embodiment 27, wherein the anti-TIM-3 antibody or the antigen-binding fragment thereof and azacitidine have the same treatment cycle, and one treatment cycle is every 1 week, every 2 weeks, every 3 weeks, or every 4 weeks; preferably, one treatment cycle is every 4 weeks.
Embodiment 29. The use according to embodiment 27, wherein the anti-TIM-3 antibody or the antigen-binding fragment thereof and decitabine have the same treatment cycle, and one treatment cycle is every 1 week, every 2 weeks, every 3 weeks, or every 4 weeks; preferably, one treatment cycle is every 4 weeks.
Embodiment 30. The use according to any one of embodiments 20-29, wherein the anti-TIM-3 antibody or the antigen-binding fragment thereof is administered at a dose of 100-1800 mg, 600-1800 mg, 600-1600 mg, or 800-1600 mg of the anti-TIM-3 antibody or the antigen-binding fragment thereof each time in an administration regimen of one administration within a single treatment cycle.
Embodiment 31. The use according to any one of embodiments 20-30, wherein the azacitidine is administered once daily at a dose of 12-100 mg/m² or 37-75 mg/m² of azacitidine each time in an administration regimen of 7-day administration within a single treatment cycle.
Embodiment 32. The use according to any one of embodiments 20-30, wherein the azacitidine is administered once daily at a dose of 200 mg or 300 mg of azacitidine each time in an administration regimen of 14-day administration within a single treatment cycle.
Embodiment 33. The use according to any one of embodiments 20-30, wherein the decitabine is administered once daily at a dose of 20 mg/m² of decitabine each time in an administration regimen of consecutively 5-day administration within a single treatment cycle.
Embodiment 34. The use according to any one of embodiments 20-30, wherein the decitabine is administered three times daily at a dose of 11-15 mg/m² of decitabine each time in an administration regimen of consecutively 3-day administration within a single treatment cycle.
Embodiment 35. A kit for use in treating a tumor, comprising the pharmaceutical combination according to any one of embodiments 1-19.
Embodiment 36. An anti-TIM-3 antibody or an antigen-binding fragment thereof, wherein the anti-TIM-3 antibody or the antigen-binding fragment thereof comprises: HCDR1 of the amino acid sequence set forth in SEQ ID NO: 1 or 11, HCDR2 of the amino acid sequence set forth in SEQ ID NO: 2 or 12, HCDR3 of the amino acid sequence set forth in SEQ ID NO: 3 or 13, LCDR1 of the amino acid sequence set forth in SEQ ID NO: 4 or 14, LCDR2 of the amino acid sequence set forth in SEQ ID NO: 5 or 15, and LCDR3 of the amino acid sequence set forth in SEQ ID NO: 6 or 16.
Embodiment 37. The anti-TIM-3 antibody or the antigen-binding fragment thereof according to embodiment 36, wherein the anti-TIM-3 antibody or the antigen-binding fragment thereof comprises: HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6.
Embodiment 38. The anti-TIM-3 antibody or the antigen-binding fragment thereof according to embodiment 36, wherein the anti-TIM-3 antibody or the antigen-binding fragment thereof comprises: HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16.
Embodiment 39. The anti-TIM-3 antibody or the antigen-binding fragment thereof according to any one of embodiments 36-38, wherein the anti-TIM-3 antibody or the antigen-binding fragment thereof comprises a heavy chain variable region having an amino acid sequence having at least 80% identity to the amino acid sequence set forth in SEQ ID NO: 7 or 17, and a light chain variable region having an amino acid sequence having at least 80% identity to the amino acid sequence set forth in SEQ ID NO: 8 or 18.
Embodiment 40. The anti-TIM-3 antibody or the antigen-binding fragment thereof according to embodiment 39, wherein the anti-TIM-3 antibody or the antigen-binding fragment thereof comprises a heavy chain variable region having an amino acid sequence having at least 80% identity to the amino acid sequence set forth in SEQ ID NO: 7, and a light chain variable region having an amino acid sequence having at least 80% identity to the amino acid sequence set forth in SEQ ID NO: 8.
Embodiment 41. The anti-TIM-3 antibody or the antigen-binding fragment thereof according to embodiment 39, wherein the anti-TIM-3 antibody or the antigen-binding fragment thereof comprises a heavy chain variable region having an amino acid sequence having at least 80% identity to the amino acid sequence set forth in SEQ ID NO: 17, and a light chain variable region having an amino acid sequence having at least 80% identity to the amino acid sequence set forth in SEQ ID NO: 18.
Embodiment 42. The anti-TIM-3 antibody or the antigen-binding fragment thereof according to embodiment 39, wherein the anti-TIM-3 antibody or the antigen-binding fragment thereof comprises a heavy chain variable region of the amino acid sequence set forth in SEQ ID NO: 7, and a light chain variable region of the amino acid sequence set forth in SEQ ID NO: 8.
Embodiment 43. The anti-TIM-3 antibody or the antigen-binding fragment thereof according to embodiment 39, wherein the anti-TIM-3 antibody or the antigen-binding fragment thereof comprises a heavy chain variable region of the amino acid sequence set forth in SEQ ID NO: 17, and a light chain variable region of the amino acid sequence set forth in SEQ ID NO: 18.
Embodiment 44. The anti-TIM-3 antibody or the antigen-binding fragment thereof according to any one of embodiments 36-43, wherein the anti-TIM-3 antibody or the antigen-binding fragment thereof is a Fab fragment, a Fab' fragment, a F(ab')₂ fragment, an scFv, an Fv fragment, an Fd fragment, an Fd' fragment, or an isolated CDR region.
Embodiment 45. The anti-TIM-3 antibody or the antigen-binding fragment thereof according to any one of embodiments 36-44, wherein the anti-TIM-3 antibody or the antigen-binding fragment thereof is a chimeric antibody, a humanized antibody, or an intact human antibody.
Embodiment 46. The anti-TIM-3 antibody or the antigen-binding fragment thereof according to any one of embodiments 36-45, wherein the anti-TIM-3 antibody or the antigen-binding fragment thereof is of the IgG1, IgG2, or IgG4 isotype.
Embodiment 47. The anti-TIM-3 antibody or the antigen-binding fragment thereof according to any one of embodiments 36-46, wherein the anti-TIM-3 antibody or the antigen-binding fragment thereof comprises a heavy chain having an amino acid sequence having at least 80% identity to the amino acid sequence set forth in SEQ ID NO: 9 or 19, and a light chain having an amino acid sequence having at least 80% identity to the amino acid sequence set forth in SEQ ID NO: 10 or 20.
Embodiment 48. The anti-TIM-3 antibody or the antigen-binding fragment thereof according to embodiment 47, wherein the anti-TIM-3 antibody or the antigen-binding fragment thereof comprises a heavy chain having an amino acid sequence having at least 80% identity to the amino acid sequence set forth in SEQ ID NO: 9, and a light chain having an amino acid sequence having at least 80% identity to the amino acid sequence set forth in SEQ ID NO: 10.
Embodiment 49. The anti-TIM-3 antibody or the antigen-binding fragment thereof according to embodiment 47, wherein the anti-TIM-3 antibody or the antigen-binding fragment thereof comprises a heavy chain having an amino acid sequence having at least 80% identity to the amino acid sequence set forth in SEQ ID NO: 19, and a light chain having an amino acid sequence having at least 80% identity to the amino acid sequence set forth in SEQ ID NO: 20.
Embodiment 50. The anti-TIM-3 antibody or the antigen-binding fragment thereof according to embodiment 47, wherein the anti-TIM-3 antibody or the antigen-binding fragment thereof comprises a heavy chain of the amino acid sequence set forth in SEQ ID NO: 9, and a light chain of the amino acid sequence set forth in SEQ ID NO: 10.
Embodiment 51. The anti-TIM-3 antibody or the antigen-binding fragment thereof according to embodiment 47, wherein the anti-TIM-3 antibody or the antigen-binding fragment thereof comprises a heavy chain of the amino acid sequence set forth in SEQ ID NO: 19, and a light chain of the amino acid sequence set forth in SEQ ID NO: 20.
Embodiment 52. The anti-TIM-3 antibody or the antigen-binding fragment thereof according to any one of embodiments 36-51, wherein the anti-TIM-3 antibody or the antigen-binding fragment thereof has at least one characteristic selected from the group consisting of: (a) binding to human TIM-3; (b) binding to monkey TIM-3; (d) stimulating IFN-γ secretion in PBMC; (e) having no ADCC; and (f) having no CDC.
Embodiment 53. An isolated nucleic acid molecule encoding the anti-TIM-3 antibody or the antigen-binding fragment thereof according to any one of embodiments 36-52.
Embodiment 54. An expression vector comprising the nucleic acid molecule according to embodiment 53.
Embodiment 55. A host cell comprising the nucleic acid molecule according to embodiment 53 or the expression vector according to embodiment 54.
Embodiment 56. A pharmaceutical composition comprising the anti-TIM-3 antibody or the antigen-binding fragment thereof according to any one of embodiments 36-52 and one or more pharmaceutically acceptable carriers.
Embodiment 57. Use of the anti-TIM-3 antibody or the antigen-binding fragment thereof according to any one of embodiments 36-52 for preparing a medicament for use in treating a tumor.
Embodiment 58. The use according to embodiment 57, wherein the tumor is a solid tumor or a non-solid tumor. Embodiment 59. The use according to embodiment 57, wherein the tumor is selected from the group consisting of brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and lymphoma.
Embodiment 60. The use according to embodiment 59, wherein the hematological malignancy is selected from the group consisting of myeloma, leukemia, myelodysplastic syndrome, myelofibrosis, and B-cell malignancy.

### Technical effects

Generally, use of the pharmaceutical combination of the present disclosure will provide:
(1) better efficacy in controlling tumor growth or even eliminating tumors as compared with either drug of the combination administered alone;
(2) fewer doses as compared with either drug of the combination administered alone;
(3) good tolerability in patients, and fewer adverse effects and/or complications as compared with either drug administered alone;
(4) a higher disease control rate in patients treated;
(5) a prolonged survival (e.g., median survival time, progression-free survival, or overall survival) in patients treated;
(6) a prolonged survival (e.g., median survival time, progression-free survival, or overall survival) in patients treated as compared with standard chemotherapies;
(7) a prolonged duration of response (DOR); and/or
(8) better activity in treating a tumor and better anti-tumor synergistic effect, as compared with either drug of the combination administered alone.

The pharmaceutical combinations and treatment regimens of the present disclosure have relatively good efficacy in treating tumors, especially hematological malignancies, with beneficial effects in at least one aspect of ORR, DCR, DOR, PFS, OS, tolerability, and side effects.

### Definitions and explanations

Unless otherwise stated, the following terms used in the present disclosure shall have the following meanings. A certain term, unless otherwise specifically defined, should not be considered uncertain or unclear, but construed according to its common meaning in the art. When referring to a trade name in the present disclosure, it is intended to refer to its corresponding commercial product or its active ingredient.

As used herein, the term "pharmaceutical combination" refers to a combination of two or more active ingredients (including administration in the form of the respective active ingredients themselves, or in the form of their respective derivatives (such as pharmaceutically acceptable salts or esters), prodrugs, or compositions) that are administered simultaneously or sequentially. The active ingredients can be administered to a subject simultaneously or sequentially in any order as individual formulations.

As used herein, the term "antibody" refers to a binding protein having at least one antigen-binding domain. The antibody and the antigen-binding fragment thereof of the present disclosure can be an intact antibody or any fragment of the antibody. Thus, the antibody and the antigen-binding fragment thereof of the present disclosure include a monoclonal antibody or a fragment thereof and an antibody variant or a fragment thereof, as well as an immunoconjugate. Examples of the antibody fragment include a Fab fragment, a Fab' fragment, a F(ab)'₂ fragment, an Fv fragment, an Fd fragment, an Fd' fragment, an isolated CDR region, a single-chain Fv molecule (scFv), and other antibody fragments known in the art, as well as antibodies subjected to any modifications known in the art (e.g., glycosylation, chemical modification, and the like). The antibody and the antigen-binding fragment thereof may also include a recombinant polypeptide, a fusion protein, a bispecific antibody, and a multispecific antibody. The anti-TIM-3 antibody and the antigen-binding fragment thereof disclosed herein may be of the IgG1, IgG2, IgG3, or IgG4 isotype. The term "isotype" refers to the class of antibodies encoded by the heavy chain constant region gene. The antibody and the antigen-binding fragment thereof may be a chimeric antibody, a humanized antibody, or an intact human antibody.

A "chimeric antibody" refers to an antibody having at least a portion of the heavy chain variable region and a portion of the light chain variable region derived from one species, and at least a portion of the constant region derived from another species. For example, in one embodiment, the chimeric antibody may comprise a murine variable region and a human constant region.

A "humanized antibody" refers to an antibody comprising complementarity determining regions (CDRs) derived from a non-human antibody, and framework and constant regions derived from a human antibody. For example, the anti-TIM-3 antibody may comprise CDRs derived from one or more murine antibodies and human framework and constant regions. Exemplary humanized antibodies are provided herein. Additional anti-TIM-3 antibodies or variants thereof comprising the HCDRs and LCDRs provided herein can be produced using any human framework sequences, and are also included in the present disclosure. In one embodiment, framework sequences suitable for use in the present disclosure include those similar in structure to the framework sequences provided herein. Additional modifications may be made in the framework regions to improve the properties of the antibodies provided herein. Such additional framework modifications may include chemical modifications, point mutations for reducing immunogenicity or removing T cell epitopes, or modifications reverting the mutations to residues in original germline sequences. In some embodiments, such modifications include those corresponding to the mutations exemplified herein, including back mutations to germline sequences. For example, in one embodiment, one or more amino acids in the human framework regions of the heavy chain variable region (VH) and/or the light chain variable region (VL) of the humanized antibodies disclosed herein are reverted to the corresponding amino acids in the parent murine antibodies.

An "antigen-binding fragment" refers to a fragment that retains the antigen-binding function of a full-length antibody, including a Fab, a Fab', a F(ab')₂, an scFv, an Fv, an Fd, an Fd', an isolated CDR, a single-domain V_{H}H fragment, and other antibody fragments known in the art, or a modified fragment by any modification method known in the art based on the above fragments. Furthermore, a recombinant polypeptide, a fusion protein, and an immunoconjugate comprising the antigen-binding fragment are also encompassed within the term antigen-binding fragment.

The term "identity" is also known as homology. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. Alignment of amino acid sequences for identity can be performed in a variety of ways within the skill in the art, e.g., BLAST, BLAST-2, ALIGN, or Megalign (DNASTAR) software. Those skilled in the art can determine suitable parameters for aligning sequences, including any algorithms required to obtain maximum alignment for the full length of sequences being compared. The term "treating" or "treatment" refers to an attempt to alter the natural course of a disease in a treated individual, and may be a clinical intervention performed for prophylaxis or during the course of clinical pathology. Desired therapeutic effects include, but are not limited to, preventing the occurrence or recurrence of diseases, relieving the symptoms of diseases, reducing any direct or indirect pathological outcomes of diseases, preventing the metastasis of diseases, slowing the progression rates of diseases, improving or alleviating the conditions of diseases, prolonging the frequency and duration of the asymptomatic period, and regressing or improving the prognosis of diseases.

The term "therapeutically effective amount" refers to an amount of the compound of the present disclosure for (i) treating a specific disease, condition, or disorder, (ii) alleviating, ameliorating, or eliminating one or more symptoms of a specific disease, condition, or disorder, or (iii) preventing or delaying the onset of one or more symptoms of the specific disease, condition, or disorder described herein. The amount of active substance (e.g., the antibody or the antigen-binding fragment thereof, or the compound of the present disclosure) constituting the "therapeutically effective amount" may vary according to factors such as the disease state, age, sex, and weight of the individual, and the ability of a therapeutic agent or a combination of therapeutic agents to elicit a desired response in the individual. The therapeutically effective amount may also be determined routinely by those skilled in the art following their knowledge and the present disclosure.

The terms "administer", "administration", or "administering" refers to physically introducing a medicament comprising a therapeutic agent to an entity using any of a variety of methods and delivery systems known to those skilled in the art.

Routes of administration of an antibody or an antigen-binding fragment thereof (e.g., the anti-TIM-3 antibody or the antigen-binding fragment thereof), or a demethylating drug (e.g., azacitidine or decitabine) include intravenous, intramuscular, subcutaneous, intraperitoneal, spinal, or other parenteral routes of administration, e.g., by injection or infusion. As used herein, the phrase "parenteral administration" refers to modes of administration apart from enteral administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. The administration may also be performed, e.g., once, multiple times, and/or over one or more extended periods of time.

The amount of the demethylating drug (e.g., azacitidine or decitabine) administered may be determined by the severity of the disease, the response to the disease, any treatment-related toxicities, and the age and health of the patient. For example, in some embodiments, the single administration dose of azacitidine may be 12-100 mg/m² or 37-75 mg/m². In some embodiments, the single administration dose of azacitidine may be about 12 mg/m² (e.g., about 12.375 mg/m²), about 19 mg/m² (e.g., about 18.75 mg/m²), about 25 mg/m² (e.g., about 24.75 mg/m² or about 25.125 mg/m²), about 28 mg/m² (e.g., about 28.125 mg/m²), about 38 mg/m² (e.g., about 37.5 mg/m²), about 50 mg/m² (e.g., about 50.25 mg/m²), about 56 mg/m² (e.g., about 56.25 mg/m²), about 75 mg/m², or about 100 mg/m² of azacitidine. In some embodiments, the single administration dose of azacitidine may be 200 mg or 300 mg. In some embodiments, the single administration dose of azacitidine may be about 75 mg/m². For another example, in some embodiments, the single administration dose of decitabine may be 11-15 mg/m². In some embodiments, the single administration dose of decitabine may be 11 mg/m² or 15 mg/m². In some embodiments, the single administration dose of decitabine may be 20-45 mg/m². In some embodiments, the single administration dose of decitabine may be 20 mg/m². In some embodiments, the daily dose of decitabine may be 33-45 mg/m². In some embodiments, the daily dose of decitabine may be 33 mg/m² or 45 mg/m². In some embodiments, the daily dose of decitabine may be 20 mg/m².

The administration regimen for a demethylating drug (e.g., azacitidine or decitabine) may be determined comprehensively depending on the activity and toxicity of the drug, the tolerance of the patient, etc. In some embodiments, azacitidine is administered once daily. In some embodiments, decitabine is administered once daily. In some embodiments, decitabine is administered three times daily. In some embodiments, azacitidine is administered intermittently, for example, 7-day administration and then 21-day interruption, or 14-day administration and then 14-day interruption. The intermittent administration mode may be repeated a plurality of times. In some embodiments, decitabine is administered intermittently, for example, 5-day administration and then 23-day interruption, or 3-day administration and then 39-day interruption. The intermittent administration mode may be repeated a plurality of times. The intermittent administration comprises an administration period and an interruption period. In the administration period, the administration can be performed once or multiple times daily. In some embodiments, azacitidine is administered once daily by subcutaneous injection at a dose of about 12 mg/m² (e.g., about 12.375 mg/m²), about 19 mg/m² (e.g., about 18.75 mg/m²), about 25 mg/m² (e.g., about 24.75 mg/m² or about 25.125 mg/m²), about 28 mg/m² (e.g., about 28.125 mg/m²), about 38 mg/m² (e.g., about 37.5 mg/m²), about 50 mg/m² (e.g., about 50.25 mg/m²), about 56 mg/m² (e.g., about 56.25 mg/m²), about 75 mg/m², or about 100 mg/m² of azacitidine each time for consecutively 7-day administration and then 21-day interruption. In some embodiments, azacitidine is administered once daily by subcutaneous injection at a dose of 37-75 mg/m² of azacitidine each time for consecutively 7-day administration and then 21-day interruption. In some embodiments, azacitidine is administered once daily by subcutaneous injection at a dose of about 75 mg/m² of azacitidine each time for consecutively 7-day administration and then 21-day interruption. In other embodiments, azacitidine is administered orally once daily at a dose of 200 mg or 300 mg of azacitidine each time for consecutively 14-day administration and then 14-day interruption. In other embodiments, azacitidine is administered orally once daily at a dose of 300 mg of azacitidine each time for consecutively 14-day administration and then 14-day interruption. In some embodiments, decitabine is administered once daily by intravenous injection at a dose of 20 mg/m² of decitabine each time for consecutively 5-day administration and then 23-day interruption. In some embodiments, decitabine is administered three times daily by intravenous injection at a dose of 11-15 mg/m², 11 mg/m², or 15 mg/m² of decitabine each time for consecutively 3-day administration and then 39-day interruption.

The term "flat dose" refers to a dose administered to a patient without considering the weight or the body surface area (BSA) of the patient. Thus, the flat dose is specified as the absolute amount of a medicament (e.g., anti-TIM-3 antibody or antigen-binding fragment thereof) rather than the mg/kg dose. For example, a 60 kg human and a 100 kg human will receive the same dose of antibody (e.g., 800 mg of anti-TIM-3 antibody or antigen-binding fragment thereof).

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutical composition" refers to a mixture consisting of one or more of the active ingredients or the pharmaceutical combinations thereof of the present disclosure and a pharmaceutically acceptable carrier. The pharmaceutical composition is intended to facilitate the administration of the compound or the pharmaceutical combination thereof of the present disclosure to a subject. As used herein, the terms "pharmaceutical composition" and "formulation" have the same meaning and are used interchangeably.

The terms "subject", "patient" and "entity" are used interchangeably herein. A "subject", "patient", or "entity" includes any human or non-human animal. The term "non-human animal" includes, but is not limited to, vertebrates such as non-human primates, sheep, dogs, and rodents such as mice, rats, and guinea pigs. In some embodiments, the term "subject", "patient", or "entity" is a mammal. In some embodiments, the subject, patient, or entity is a mouse. In some embodiments, the subject, patient, or entity is a human being.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to ± 5%, for example, fluctuating within a particular numerical range given ± 2%, ± 1%, or ± 0.5%. Where a particular value is given in the present disclosure or in the claims, unless otherwise stated, "about" should be considered to mean being within an acceptable error range for that particular value. In this context, unless otherwise stated, the values of the parameters or conditions in a step are all modified by "about" by default.

As used herein, "combined use" or "use in combination" means that two or more active substances may be administered to a subject simultaneously or sequentially in any order as individual formulations.

The term "single dose" refers to the smallest unit of packaging containing a certain quantity of a pharmaceutical product, for example, a box of seven tablets, one tablet being a single dose; or a vial of injection is a single dose. The term "multiple doses" consists of multiple single doses. A "unit dose" refers to the dose of active ingredient contained in the smallest unit of packaging containing a certain quantity of a pharmaceutical product, e.g., the dose of azacitidine contained in one azacitidine tablet is a unit dose; or the dose of an antibody contained in one vial of the antibody injection is a unit dose.

The term "fixed combination" refers to administration of the active components (e.g., the anti-TIM-3 antibody or the antigen-binding fragment thereof, azacitidine or decitabine) to a subject simultaneously at a fixed total dose or in a fixed dose proportion, or in the form of a single substance, pharmaceutical composition or formulation.

The term "non-fixed combination" refers to simultaneous, parallel, or sequential and temporally unlimited administration of two or more active components as independent substances (for example, a pharmaceutical composition and a formulation) to a subject, wherein the active ingredients administered to the subject reach therapeutically effective amounts. An enumerable example of the non-fixed combination is cocktail therapy, for example, 3 or more active components are administered. In a non-fixed combination, each of the active components can be packaged, sold, or administered as a fully independent pharmaceutical composition. The term "non-fixed combination" also includes the combined use of "fixed combinations", or a "fixed combination" and an independent entity of any one or more active components.

The term "recurrent" cancer is one that regenerates at an initial site or a distant site after being responsive to initial treatment (e.g., surgery). A "locally recurrent" cancer is one that occurs, after treatment, at the same location as the previously treated cancer.

The term "refractory" refers to the situation where a subject or mammal has residual cancer cells in its body even after intensive treatment.

The term "metastatic" cancer refers to one that spreads from one part of the body (e.g., the lung) to another part of the body.

As used herein, "treatment failure" is defined as the occurrence of disease progression or recurrence during the treatment or after the last treatment, or intolerance due to toxic side effects during the treatment.

Unless otherwise specified clearly herein, singular terms encompass plural referents, and vice versa.

All patents, patent applications and other identified publications are explicitly incorporated herein by reference for the purpose of description and disclosure. These publications are provided solely because they were disclosed prior to the filing date of the present disclosure. All statements as to the dates of these documents or descriptions as to the contents of these documents are based on the information available to the applicant and do not constitute any admission as to the correctness of the dates or the content of these documents. Moreover, in any country or region, any reference to these publications herein shall not be construed as an admission that the publications form part of the common knowledge in the art.

### Example

For clarity, the present disclosure is further described with the following examples, which are, however, not intended to limit the scope of the present disclosure. All the reagents used in the present disclosure are commercially available and can be used without further purification.

The mouse antibody mAb 50B5 was chosen for humanization design. The mouse antibody mAb 50B5 is the anti-TIM-3 antibody mAb 50B5 described in the patent application document with the publication number WO2020041520 or CN112566936, and the entire contents of the patent application documents WO2020041520 and CN112566936 are incorporated herein by reference.

A human germline antibody framework region that could be used for humanization of the mouse antibody was screened using the CDR graft method. The CDRs of the mouse antibody were inserted into the selected framework regions to obtain a humanized antibody by screening a human germline antibody or a subtype thereof having the highest sequence identity to the amino acid sequence of the variable region of the mouse antibody. The amino acid sequences of the heavy chain variable region and the light chain variable region of the humanized antibody are set forth in SEQ ID NO: 7 and SEQ ID NO: 8, respectively.

A VL gene synthetic fragment of the humanized antibody was ligated to a human light chain constant region to construct a humanized light chain, and a VH gene synthetic fragment of the humanized antibody was ligated to a human IgG4 heavy chain constant region to construct a humanized heavy chain. The vector containing the humanized light chain DNA and the vector containing the humanized heavy chain DNA were transfected into Expi-CHO cells for protein expression, and then the humanized antibody in cell culture supernatant was purified by a Protein A column to obtain a humanized anti-TIM-3 antibody h50B5, whose heavy chain amino acid sequence is set forth in SEQ ID NO: 9, and light chain amino acid sequence is set forth in SEQ ID NO: 10. The examples of the present disclosure adopted the anti-TIM-3 antibody TSR022 as a benchmark. The anti-TIM-3 antibody TSR022 was prepared by referring to the aforementioned method, with its heavy chain amino acid sequence set forth in SEQ ID NO: 22 and its light chain amino acid sequence set forth in SEQ ID NO: 23.

EVQLLESGGGLVQPGGSLRLSCAAASGFTFSSYDMSWVRQAPGKGLDWVSTISGGGTYTYYQDSVKGR
FTISRDNSKNTLYLQMNSLRAEDTAVYYCASMDYWGQGTTVTVSSASTKGPSVFPLAPCSRSTSESTAAL
GCLVKDYFPEPVTVSWNSGALTSGVHTFPAVLQSSGLYSLSSVVTVPSSSLGTKTYTCNVDHKPSNTKVD
KRVESKYGPPCPPCPAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKGQPREPQVYTLPPSQ
EEMTKNQVSLTCLVKGFYPSDIAVEWESNGQPENNYKTTPPVLDSDGSFFLYSRLTVDKSRWQEGNVFS
CSVMHEALHNHYTQKSLSLSLGK (SEQ ID NO:22);

DIQMTQSPSSLSASVGDRVTITCRASQSIRRYLNWYHQKPGKAPKLLIYGASTLQSGVPSRFSGSGSGTDF
TLTISSLQPEDFAVYYCQQSHSAPLTFGGGTKVEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNR
GEC (SEQ ID NO:23).

The examples of the present disclosure used human IgG4 (hIgG4) (prepared in-house) as a negative control.

### Example 1: Binding of Anti-TIM-3 Antibodies to TIM-3 Recombinant Proteins of Different Species and Non-TIM-3 Recombinant Proteins

Human TIM-3 (R&D), mouse TIM-3 (Novoprotein), rat TIM-3 (Chinese Academy of Sciences Shanghai Institute of Materia Medica), human B7-1 (Sino Biological Inc.), human B7-H3 (Sino Biological Inc.), human PD-1 (Sino Biological Inc.), or human EGFR (Sino Biological Inc.) recombinant proteins were coated in an ELISA 96-well plate (Greiner), and different concentrations of the anti-TIM-3 antibody h50B5 (100000 ng/mL, 30000 ng/mL, 10000 ng/mL, 3000 ng/mL, 1000 ng/mL, 300 ng/mL, 100 ng/mL, 30 ng/mL, 10 ng/mL, and 3 ng/mL) were added to the 96-well plate for incubation at 37 °C for 1 h. After the 96-well plate was washed with PBS (pH 7.4), Goat anti-human Ig kappa chain-HRP conjugate (Millipore) was added for incubation at 37 °C for 1 h, and the HRP substrate o-phenylenediamine dihydrochloride (OPD) was added. After the reaction was stopped with H₂SO₄, the OD value was measured at a wavelength of 450 nm using a full-function microplate reader Synergy H4 (BioTek), and the binding EC₅₀ was calculated based on the OD value.

In the binding experiment to cynomolgus monkey TIM-3 recombinant protein, h50B5 was coated in an ELISA 96-well plate (Greiner) at a concentration of 2 µg/mL, and different concentrations of cynomolgus monkey TIM-3 recombinant protein (Acro) were added (starting at 16 µg/mL, 4-fold gradient dilution, a total of 10 concentrations) for incubation at room temperature for 2 h. After the 96-well plate was washed with PBST for 3 times, Anti-His HRP (Sino Biological Inc.) was added for incubation at room temperature for 1 h, and the HRP substrate OPD was added. After the reaction was stopped with H₂SO₄, the OD value was measured at a wavelength of 450 nm using a full-function microplate reader Synergy H4 (BioTek), and the binding EC₅₀ was calculated based on the OD value. The results showed that the EC₅₀ of h50B5 binding to human TIM-3 protein was 291.0 ± 14.1 ng/mL, with a maximum binding Eₘₐₓ (OD₄₅₀) of 0.52 ± 0.03; and the EC₅₀ of h50B5 binding to cynomolgus monkey TIM-3 protein was 1639.0 ± 14.1 ng/mL, with a maximum binding Eₘₐₓ (OD₄₅₀) of 0.34 ± 0.03. At 100 µg/mL, h50B5 bound to both human and cynomolgus monkey TIM-3 proteins, and the binding amount to human TIM-3 protein was the highest; there is no obvious binding to mouse and rat TIM-3 proteins, indicating that h50B5 had species selectivity; and there is no obvious binding to human B7-1, human B7-H3, human PD-1, and human EGFR proteins, indicating that the binding of h50B5 to human TIM-3 is specific.

### Example 2: Binding of Anti-TIM-3 Antibodies to Cells Highly Expressing Human TIM-3

The human TIM-3 gene was transfected into CHO-S cells, and the cells were incubated in an incubator at 37 °C with 5% CO₂ using a Dynamis AGT medium (containing 4 mM glutamic acid and 3 µg/mL Puromycin) to give CHO-S-HuTIM-3 cells. Different concentrations of h50B5 or TSR022 (300000 ng/mL, 100000 ng/mL, 30000 ng/mL, 10000 ng/mL, 3000 ng/mL, 1000 ng/mL, 300 ng/mL, 100 ng/mL, 30 ng/mL, and 10 ng/mL) were incubated with CHO-S-HuTIM-3 cells at 4 °C for 1 h, followed by addition of Goat anti-human Ig kappa chain-HRP conjugate for incubation at 37 °C for 1 h, and finally the HRP substrate OPD was added. After the reaction was stopped with H₂SO₄, the OD value was measured at a wavelength of 450 nm using a full-function microplate reader Synergy H4. The binding EC₅₀ was calculated based on the OD value.

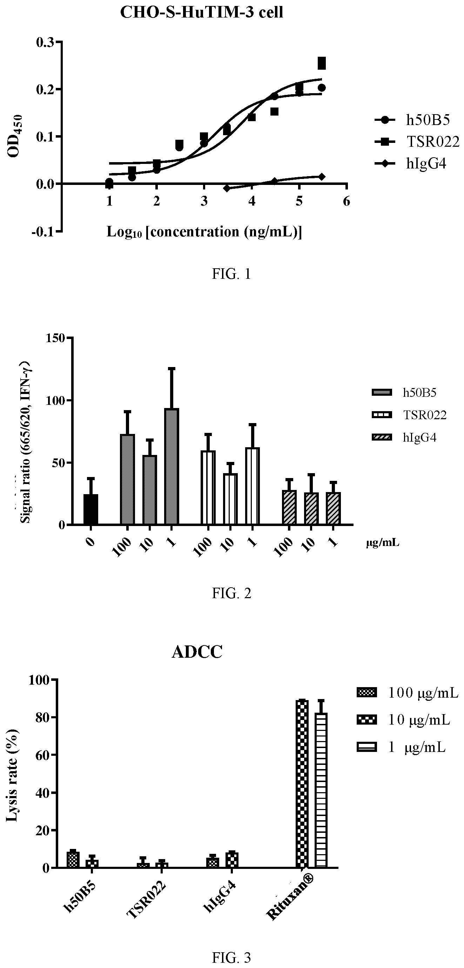

As shown in FIG. 1, h50B5 had strong binding affinity for CHO-S-HuTIM-3 cells highly expressing human TIM-3, with an EC₅₀ and maximum binding Eₘₐₓ (OD₄₅₀) of 1456.0 ± 338.0 ng/mL and 0.21 ± 0.03, respectively; and TSR022 had an EC₅₀ and maximum binding Eₘₐₓ (OD₄₅₀) of 6815.5 ± 166.2 ng/mL and 0.26 ± 0.05, respectively, when binding to CHO-S-HuTIM-3 cells. This indicated that h50B5 had a higher binding activity than that of TSR022.

### Example 3: Effect of Anti-TIM-3 Antibodies on IFN-γ Secretion in Mixed Lymphocyte Reaction (MLR)

After CD14⁺ monocytes were sorted from human PBMCs (prepared in-house) using a CD14 magnetic bead sorting kit (MiltenyiBiotec), the cells were induced with GM-CSF (Xiamen Amoytop Biotech Co., Ltd.) for 7 days to differentiate into mature dendritic cells (DCs); and after CD4⁺ T cells were sorted from human PBMCs using a CD4⁺ T cell magnetic bead sorting kit (Miltenyi Biotec), the CD4⁺ T cells were mixed with the mature DCs and added different concentrations of h50B5 or TSR022 (100 µg/mL, 10 µg/mL, and 1 µg/mL), with a blank control (no antibody added) set up, for incubation for 5 days. After the incubation was completed, the supernatant was aspirated, and the content of IFN-γ in the supernatant was detected by using an IFN-γ detection kit (Cisbio).

As shown in FIG. 2, mature dendritic cells interacted with heterologous CD4⁺ T cells, and h50B5 was capable of significantly promoting the secretion of IFN-γ in MLR, showing greater activity than that of TSR022.

### Example 4: Antibody-Dependent Cell-Mediated Cellular Cytotoxicity (ADCC) of Anti-TIM-3 Antibodies

CHO-S-HuTIM-3 and Daudi cells (Shanghai Cell Bank of Chinese Academy of Sciences) were labeled with Calcein-AM dye (Dojindo Laboratories), and mixed with 10 µg/mL or 100 µg/mL of h50B5 (with CHO-S-huTIM-3 cells as the target cells), 10 µg/mL or 100 µg/mL of TSR022 (with CHO-S-huTIM-3 cells as the target cells), 10 µg/mL or 100 µg/mL of hIgG4 (with CHO-S-huTIM-3 cells as the target cells), or 1 or 10 µg/mL of Rituxan^{®} (with Daudi cells as the target cells), with a blank control (no antibody added) set up, and reacted for 1 h, and then effector cells PBMC (Changhai Hospital of Shanghai) were added and reacted for 4 h. After the reaction was completed, the mixture was centrifuged, and the supernatant was collected and measured for fluorescence values at 490 nm as excitation wavelength and 515 nm as emission wavelength. The cells were lysed directly with 0.1% Triton X-100 and centrifuged, and then the supernatant was collected to determine the total fluorescence value. The lysis rate (%) was calculated based on the fluorescence value.

As shown in FIG. 3, Rituxan^{®} exhibited significant ADCC against Daudi cells, while h50B5 and TSR022 did not show significant ADCC against CHO-S-HuTIM-3 cells.

### Example 5: Complement-Dependent Cytotoxicity (CDC) of Anti-TIM-3 Antibodies

CHO-S-HuTIM-3 cells and Daudi cells (Shanghai Cell Bank of Chinese Academy of Sciences) were inoculated into a 96-well plate. 10 µg/mL or 100 µg/mL of h50B5 (with CHO-S-huTIM-3 cells as the target cells), 10 µg/mL or 100 µg/mL of TSR022 (with CHO-S-huTIM-3 cells as the target cells), or 10 µg/mL or 100 µg/mL of Rituxan^{®} (with Daudi cells as the target cells) was added and reacted for 1 h, and then 10% (v/v) of human serum was added and reacted for 4 h. Finally, CCK-8 reagent (Dojindo Laboratories) was added and reacted for 4 h. The OD value was measured at a wavelength of 450 nm using a microplate reader, and the lysis rate (%) was calculated.

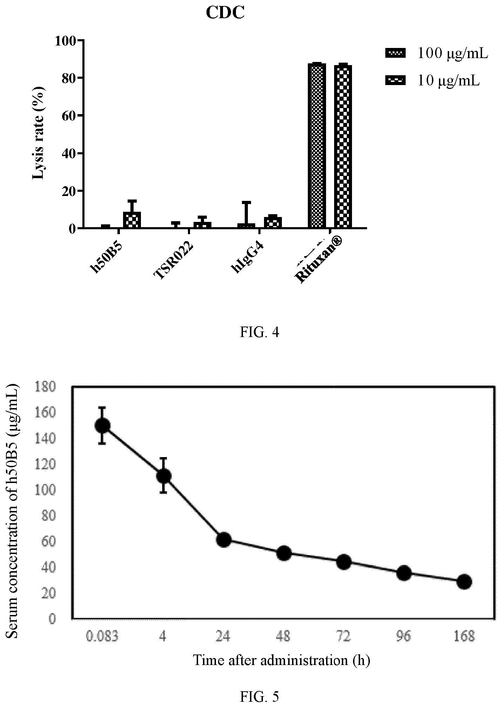

As shown in FIG. 4, Rituxan^{®} exhibited significant CDC against Daudi cells, while h50B5 and TSR022 did not show significant CDC against CHO-S-HuTIM-3 cells.

### Example 6: Effect of Anti-TIM-3 Antibodies on Tumor-Infiltrating Lymphocytes of Lung Cancer HCC827 Subcutaneous Xenograft Tumors in Human Immune Reconstituted Mice (PBMC-NCG)

NCG mice were subcutaneously inoculated with HCC827 cells (Cell Bank of Chinese Academy of Sciences) at 8 × 10⁶ cells/mouse. When the tumor volume reached 100-150 mm³, human PBMC (Allcells Biotechnology (Shanghai) Co., Ltd., Cat. No.: PB004F) was injected intravenously at 6 × 10⁶ cells/mouse. Intravenous injection of h50B5 or hIgG4 (administration dose of 10 mg/kg) was performed the next day, blood was collected from the mouse orbits at different time points (5 min, 4 h, 24 h, 48 h, 72 h, 96 h, and 168 h) after the administration, and serum was isolated to detect drug concentration. Orbital blood was collected from 6 mice at each time point. 72 h after administration, groups 1 and 3 were dissected, and 168 h after administration, groups 2 and 4 were dissected. Tumor tissues were taken separately, tumor-infiltrating lymphocytes (TILs) were isolated, and the proportions of human CD45⁺, CD3⁺, CD4⁺, and CD8⁺ cells were analyzed by a flow cytometer. The experimental design is shown in Table 1. min represents minutes, h represents hours.

**Table 1. Experimental design**

| Group | Administration | Number of mice | Time point of blood collecting | Time to isolate TILs |
|---|---|---|---|---|
| 1 | hIgG4 10 mg/kg | 6 | 5 min, 24 h, 72 h | 72 h |
| 2 | hIgG4 10 mg/kg | 6 | 4 h, 48 h, 96 h, 168 h | 168 h |
| 3 | h50B5 10 mg/kg | 6 | 5 min, 24 h, 72 h | 72 h |
| 4 | h50B5 10 mg/kg | 6 | 4 h, 48 h, 96 h, 168 h | 168 h |

As shown in Table 2, 168 h after intravenous injection of h50B5 (10 mg/kg) to tumor-bearing mice, the ratio of CD8⁺/CD3⁺ T cells in TILs was significantly higher compared to the hIgG4 group (46.9 ± 6.9% vs 31.1 ± 6.9%, P < 0.01), and the ratio of CD8⁺/CD4⁺ T cells was also significantly higher compared to the hIgG4 group (112.7 ± 32.1% vs 60.2 ± 11.3%, P< 0.01). This indicates that h50B5 can promote infiltration of CD8⁺ T cells in the HCC827 subcutaneous xenograft tumor tissue and significantly improve the proportion of CD8⁺ T cells in CD3⁺ T cells. As shown in FIG. 5, the serum concentration of h50B5 gradually decreased over time after a single intravenous injection of h50B5 (10 mg/kg) in tumor-bearing mice. 168 h after administration, the serum concentration of h50B5 still reached 29.4 ± 2.3 µg/mL. The Cₘₐₓ was 150.3 µg/mL, and the AUC_{0-168 h} was 8142.0 h·µg·mL⁻¹.

**Table 2. Effect of a single intravenous injection of h50B5 or hIgG4 on the proportions of various T cell subsets in TILs of lung cancer HCC827 subcutaneous xenograft tumors in human immune reconstituted mice (PBMC-NCG)**

| Time after administration | Group | CD3⁺/CD45⁺ (%) | CD4⁺/CD3⁺ (%) | CD8⁺/CD3⁺ (%) | CD8⁺/CD4⁺ (%) |
|---|---|---|---|---|---|
| 72h | hIgG4 | 98.1 ± 2.4 | 55.6 ± 2.8 | 26.3 ± 3.5 | 47.5 ± 7.8 |
| | h50B5 | 97.7 ± 1.1 | 52.7 ± 5.3 | 29.3 ± 3.9 | 56.8 ± 14.1 |
| 168 h | hIgG4 | 98.3 ± 1.4 | 51.6 ± 5.6 | 31.1 ± 6.9 | 60.2 ± 11.3 |
| | h50B5 | 97.5 ± 2.5 | 43.3 ± 8.0 | 46.9 ± 6.9** | 112.7 ± 32.1** |

| | | | | | |
|---|---|---|---|---|---|
| Mean ± SD; **p < 0.01 vs hIgG4 group. | | | | | |

### Example 7: Safety Study of Anti-TIM-3 Antibodies

A single intravenous injection of h50B5 to cynomolgus monkeys: During the experiment, no abnormalities were observed in any of the indexes of the cynomolgus monkeys (general state observation, body weight, food intake, body temperature, II-lead electrocardiogram, blood pressure, respiration, cytokine, complement, circulating immune complex, immunoglobulin, T lymphocyte subset, hematology examination, blood biochemical examination, urine examination, ophthalmology examination, bone marrow smear examination, gross anatomy observation, and histopathology), and no anti-h50B5 antibodies were detected in the serum of the cynomolgus monkeys. The maximum tolerated dose (MTD) of a single intravenous injection of h50B5 in the cynomolgus monkeys was 500 mg/kg.

Multiple intravenous injections of h50B5 to cynomolgus monkeys (intravenous injections of 15 mg/kg, 50 mg/kg, or 150 mg/kg of h50B5 to cynomolgus monkeys once a week for 4 consecutive weeks (5 total administrations)): During the experiment, no abnormalities were observed in any of the indexes of each group of the cynomolgus monkeys (general state observation, body weight, food intake, body temperature, II-lead electrocardiogram, blood pressure, respiration, cytokine, complement, circulating immune complex, immunoglobulin, T lymphocyte subset, hematology examination, blood biochemical examination, urine examination, ophthalmology examination, bone marrow smear examination, gross anatomy observation, and histopathology), and no anti-h50B5 antibodies were detected in the serum of each group of the cynomolgus monkeys. The cynomolgus monkeys received h50B5 via intravenous injection once a week for 5 consecutive times, with no observed adverse effect level (NOAEL) being 150 mg/kg.

Phase I safety, tolerability and pharmacokinetic study of h50B5 injection: Subjects with advanced solid tumors were selected for the study, and the results showed that no dose-limiting toxicity (DLT) was observed in all dose groups within the dose range of 1-1800 mg/person.

### Example 8: Clinical Trials of Recurrent/Refractory Acute Myelogenous Leukemia (AML) and Myelodysplastic Syndrome (MDS)

1. Inclusion criteria
   a) Pathologically confirmed subjects with recurrent/refractory intermediate to high risk (IPSS-R intermediate risk and above) MDS and AML who meet the criteria of the World Health Organization (WHO) Classification of Hematopoietic and Lymphoid Tissue Tumors 2016 (Revised), or subjects with MDS and AML who are intolerant to other drug treatments (for example, experiencing drug-related grade ≥3 toxicity that leads to permanent drug withdrawal during treatment), and whom the investigator judges to have no other appropriate treatment options.
   b) Age ≥ 18 years (at the time of signing the informed consent), with no gender restrictions.
   c) ECOG score: 0-2 points.
   d) Expected survival time: ≥ 3 months.
   e) The functioning of main organs is normal, i.e., meets the following criteria:
      i. Blood routine examination (no blood transfusion and no growth factor support within 7 days before examination):
         AML subjects: hemoglobin (HGB) ≥ 60 g/L; neutrophil count (NEUT) ≥ 0.5 × 10⁹/L; platelet count (PLT) ≥ 20 × 10⁹/L (in the case of low hemogram due to this disease, it is permissible to meet this criterion after receiving platelet transfusions and/or the use of hematopoietic stimulating factors); white blood cell (WBC) ≤ 30 × 10⁹/L (it is permissible to meet this criterion after receiving hydroxyurea or leukocyte apheresis).
         MDS subjects: hemoglobin (HGB) ≥ 60 g/L; neutrophil count (NEUT) ≥ 0.15 × 10⁹/L; platelet count (PLT) ≥ 20 × 10⁹/L (in the case of low hemogram due to this disease, it is permissible to meet this criterion after receiving platelet transfusions and/or the use of hematopoietic stimulating factors).
      ii. Biochemical examination of blood: alanine transaminase (ALT) and aspartate transaminase (AST) ≤ 2.5× ULN; total bilirubin (TBIL) ≤ 1.5× ULN; creatinine (CRE) ≤ 1.5× ULN or creatinine clearance rate ≥ 50 mL/min.
      iii. Blood coagulation: activated partial thromboplastin time (APTT), international normalized ratio (INR), prothrombin time (PT) ≤ 1.5× ULN.
      iv. Left ventricular ejection fraction (LVEF) ≥ 50%.
   f) Female patients of childbearing age must have a negative serum pregnancy test within 7 days before study enrollment and must be non-lactating; patients should agree to take contraceptive measures during the study and for 6 months after the study.
   g) Voluntary participation, written informed consent and good compliance.
2. Test drugs and administration regimens
   h50B5 injection: 4 weeks for one treatment cycle with h50B5 administered once on day 8 of each treatment cycle at a dose of 800 mg, 1200 mg, or 1600 mg via intravenous drip.
   Azacitidine: 4 weeks for one treatment cycle with azacitidine administered on days 1-7 of each treatment cycle via subcutaneous injection (daily dose 75 mg/m²).
   Decitabine: 4 weeks for one treatment cycle with decitabine administered on days 1-5 of each treatment cycle via intravenous infusion (daily dose 20 mg/m²).
   The h50B5 injection was used in combination with azacitidine, or the h50B5 injection was used in combination with decitabine.
3. Evaluation criteria
   The effectiveness was evaluated by a combination of bone marrow aspiration and hematology examinations, and the efficacy was evaluated according to the MDS International Working Group (IWG) 2006 Efficacy Evaluation Criteria, the AML IWG Response Criteria (Revised), and the European Leukemia Network (ELN) Guidelines.
4. Endpoint
   4.1 Objective response rate (ORR):
      At the end of the study, for MDS subjects: ORR = complete response (CR) + marrow complete response (mCR) + partial response (PR); for AML subjects: ORR = CR + complete remission with incomplete count recovery (CRi) + complete response with incomplete recovery of platelets (CRp) + morphological leukemia-free state (MLFS) + PR;
   4.2 incidence and severity of adverse event (AE);
   4.3 receptor occupancy (RO) of TIM-3 after administration;
   4.4 related indexes of immunogenicity: the incidence of anti-drug antibodies (ADA) and their titers, the incidence of neutralizing antibodies (Nab) in the subjects;
   4.5 other therapeutic indexes: progression-free survival (PFS), disease control rate (DCR), CR/mCR rate (MDS), CR/CRi rate (AML), overall survival (OS), and duration of response (DOR).
      Moreover, h50B5 treatment-related biomarkers were evaluated, such as TIM-3 expression in blood samples.
5. Therapeutic effects
   Preliminary results showed that the treatment regimen of the combination of h50B5 injection and azacitidine could safely and effectively treat AML and/or MDS, especially recurrent or refractory AML and/or MDS, and had the potential advantages of prolonging the progression-free survival (PFS) and overall survival (OS) of the subjects, thereby bringing significantly improved clinical efficacy and survival benefit to the subjects.
   After treatment with h50B5 injection (1200 mg) in combination with azacitidine, complete response (CR) (33%) was achieved in 1 out of 3 evaluable subjects (all diagnosed with AML) with a disease control rate (DCR) of 100%. A typical case for this study is as follows:
      Case: recurrent AML

The patient, a 69-year-old male, visited the hospital for "fatigue and shortness of breath" more than 3 years ago and was discharged after improvement with treatment. Later, he developed a cough and expectoration without obvious cause, more pronounced in the morning, with blood-tinged sputum, and sought medical attention. Blood routine examination showed: WBC 26.0 × 10⁹/L, NEUT 0.7 × 10⁹/L, LYMPH 5.3 × 10⁹/L, MONO 20.0 × 10⁹/L, RBC 1.90 × 10¹²/L, HGB 70 g/L, MCV 108.9 fL, MCH 36.8 pg, and PLT 6 × 10⁹/L. Bone marrow smear: characterized predominantly by primitive and immature monocyte hyperplasia, which accounted for 16%. Peripheral blood smear showed a large number of primitive and immature cells. Bone marrow flow cytometry: detected 51.08% of abnormal primitive/immature myeloid cells among nucleated cells, with a notably increased proportion of monocytes at 21.7%. The patient was evaluated for complete recurrence of leukemia.

After treatment with the administration regimen of h50B5 injection (1200 mg) in combination with azacitidine, the optimal therapeutic effect was CR according to the efficacy evaluation criteria.

## Claims

1. A pharmaceutical combination comprising an anti-TIM-3 antibody or an antigen-binding fragment thereof and a demethylating drug, wherein the anti-TIM-3 antibody or the antigen-binding fragment thereof comprises:
HCDR1 of the amino acid sequence set forth in SEQ ID NO: 1 or 11, HCDR2 of the amino acid sequence set forth in SEQ ID NO: 2 or 12, HCDR3 of the amino acid sequence set forth in SEQ ID NO: 3 or 13, LCDR1 of the amino acid sequence set forth in SEQ ID NO: 4 or 14, LCDR2 of the amino acid sequence set forth in SEQ ID NO: 5 or 15, and LCDR3 of the amino acid sequence set forth in SEQ ID NO: 6 or 16; preferably,
the anti-TIM-3 antibody or the antigen-binding fragment thereof comprises: HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6; or the anti-TIM-3 antibody or the antigen-binding fragment thereof comprises: HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16.

2. The pharmaceutical combination according to claim 1, wherein the anti-TIM-3 antibody or the antigen-binding fragment thereof comprises: a heavy chain variable region having an amino acid sequence having at least 95% identity to the amino acid sequence set forth in SEQ ID NO: 7 or 17, and a light chain variable region having an amino acid sequence having at least 95% identity to the amino acid sequence set forth in SEQ ID NO: 8 or 18; preferably,
the anti-TIM-3 antibody or the antigen-binding fragment thereof comprises: a heavy chain variable region having an amino acid sequence having at least 95% identity to the amino acid sequence set forth in SEQ ID NO: 7, and a light chain variable region having an amino acid sequence having at least 95% identity to the amino acid sequence set forth in SEQ ID NO: 8; or the anti-TIM-3 antibody or the antigen-binding fragment thereof comprises: a heavy chain variable region having an amino acid sequence having at least 95% identity to the amino acid sequence set forth in SEQ ID NO: 17, and a light chain variable region having an amino acid sequence having at least 95% identity to the amino acid sequence set forth in SEQ ID NO: 18.

3. The pharmaceutical combination according to claim 1 or 2, wherein the anti-TIM-3 antibody or the antigen-binding fragment thereof comprises: a heavy chain having an amino acid sequence having at least 95% identity to the amino acid sequence set forth in SEQ ID NO: 9 or 19, and a light chain having an amino acid sequence having at least 95% identity to the amino acid sequence set forth in SEQ ID NO: 10 or 20; preferably,
the anti-TIM-3 antibody or the antigen-binding fragment thereof comprises: a heavy chain having an amino acid sequence having at least 95% identity to the amino acid sequence set forth in SEQ ID NO: 9, and a light chain having an amino acid sequence having at least 95% identity to the amino acid sequence set forth in SEQ ID NO: 10; or the anti-TIM-3 antibody or the antigen-binding fragment thereof comprises: a heavy chain having an amino acid sequence having at least 95% identity to the amino acid sequence set forth in SEQ ID NO: 19, and a light chain having an amino acid sequence having at least 95% identity to the amino acid sequence set forth in SEQ ID NO: 20.

4. The pharmaceutical combination according to any one of claims 1-3, wherein the demethylating drug is azacitidine or decitabine.

5. The pharmaceutical combination according to any one of claims 1-4, wherein a unit dose of the anti-TIM-3 antibody or the antigen-binding fragment thereof is 60-1800 mg, 100-1600 mg, 120-1600 mg, 180-1200 mg, or 240-600 mg; optionally,
a unit dose of the azacitidine is 100 mg, 200 mg, and/or 300 mg; or a unit dose of the decitabine is 10 mg, 25 mg, and/or 50 mg.

6. The pharmaceutical combination according to any one of claims 1-5, wherein the mass ratio of the anti-TIM-3 antibody or the antigen-binding fragment thereof to azacitidine is (0.01-30):1, (0.1-15):1, (0.1-10):1, (0.1-5):1, (0.5-5):1, (0.5-3):1, (0.1-0.5):1, or (1-3):1; or
the mass ratio of the anti-TIM-3 antibody or the antigen-binding fragment thereof to decitabine is (0.1-60):1, (0.2-30):1, (2-15):1, (3-15):1, (3-12):1, or (6-12):1.

7. The pharmaceutical combination according to any one of claims 1-5, wherein the pharmaceutical combination comprises 100-1800 mg, 600-1800 mg, 600-1600 mg, or 800-1600 mg of the anti-TIM-3 antibody or the antigen-binding fragment thereof; optionally,
the pharmaceutical combination further comprises 12-100 mg/m², 37-75 mg/m², or 200-300 mg of azacitidine; or 20-45 mg/m² of decitabine.

8. The pharmaceutical combination according to any one of claims 1-5, wherein the pharmaceutical combination is suitable for administration within a single treatment cycle and comprises 100-1800 mg, 600-1800 mg, 600-1600 mg, or 800-1600 mg of the anti-TIM-3 antibody or the antigen-binding fragment thereof; optionally,
the pharmaceutical combination further comprises 86-700 mg/m², 262-525 mg/m², or 1400-4200 mg of azacitidine; or 99-135 mg/m² of decitabine.

9. Use of the pharmaceutical combination according to any one of claims 1-8 for preparing a medicament for use in treating a tumor.

10. The use according to claim 9, wherein the tumor is a solid tumor or a non-solid tumor; more preferably, the tumor is selected from the group consisting of brain cancer, head and neck cancer, esophageal cancer, pancreatic cancer, pancreatic ductal carcinoma, gastric cancer, lung cancer, lung adenocarcinoma, kidney cancer, adrenocortical carcinoma, liver cancer, bile duct cancer, gallbladder cancer, bone cancer, thymus cancer, cervical cancer, breast cancer, ovarian cancer, fallopian tube cancer, endometrial cancer, uterine cancer, vaginal cancer, vulvar cancer, prostate cancer, penile cancer, testicular cancer, urethral cancer, anal cancer, duodenal cancer, colorectal cancer, bladder cancer, melanoma, skin cancer, dermatofibrosarcoma protuberan, Merkel cell carcinoma, squamous cell carcinoma, neuroendocrine malignant tumor, glioblastoma, glioma, sarcoma, soft tissue sarcoma, mesothelioma, hematological malignancy, and lymphoma.

11. The use according to claim 10, wherein the hematological malignancy is selected from the group consisting of myeloma, leukemia, myelodysplastic syndrome, myelofibrosis, and B cell malignancy.

12. The use according to any one of claims 9-11, wherein the anti-TIM-3 antibody or the antigen-binding fragment thereof and the demethylating drug can be administered simultaneously, sequentially, and/or alternately; optionally,
the anti-TIM-3 antibody or the antigen-binding fragment thereof and azacitidine can be administered simultaneously, sequentially, and/or alternately; or the anti-TIM-3 antibody or the antigen-binding fragment thereof and decitabine can be administered simultaneously, sequentially, and/or alternately.

13. The use according to any one of claims 9-12, wherein the anti-TIM-3 antibody or the antigen-binding fragment thereof and demethylating drug have the same treatment cycle, and one treatment cycle is every 1 week, every 2 weeks, every 3 weeks, or every 4 weeks; optionally,
the anti-TIM-3 antibody or the antigen-binding fragment thereof and azacitidine have the same treatment cycle, and one treatment cycle is every 1 week, every 2 weeks, every 3 weeks, or every 4 weeks; or the anti-TIM-3 antibody or the antigen-binding fragment thereof and decitabine have the same treatment cycle, and one treatment cycle is every 1 week, every 2 weeks, every 3 weeks, or every 4 weeks.

14. The use according to any one of claims 9-13, wherein the anti-TIM-3 antibody or the antigen-binding fragment thereof is administered at a dose of 100-1800 mg, 600-1800 mg, 600-1600 mg, or 800-1600 mg of the anti-TIM-3 antibody or the antigen-binding fragment thereof each time in an administration regimen of one administration within a single treatment cycle; optionally,
the azacitidine is administered once daily at a dose of 12-100 mg/m² or 37-75 mg/m² of azacitidine each time in an administration regimen of 7-day administration within a single treatment cycle; or the azacitidine is administered once daily at a dose of 200 mg or 300 mg of azacitidine each time in an administration regimen of 14-day administration within a single treatment cycle; or the decitabine is administered once daily at a dose of 20 mg/m² of decitabine each time in an administration regimen of consecutively 5-day administration within a single treatment cycle; or the decitabine is administered three times daily at a dose of 11-15 mg/m² of decitabine each time in an administration regimen of consecutively 3-day administration within a single treatment cycle.

15. A kit for use in treating a tumor, comprising the pharmaceutical combination according to any one of claims 1-8.
